# EUROPÄISCHE PATENTSCHRIFT

(11) **EP 1 970 084 B1**
(45) Veröffentlichungstag und Bekanntmachung des Hinweises auf die Patenterteilung: **11.08.2010**
(21) Anmeldenummer: 07005216.2
(22) Anmeldetag: 14.03.2007
(51) Int. Cl.: A61M 5/158

(54) **Insertionsvorrichtung für einen Insertionskopf, insbesondere für ein Infusionsset**
Insertion device for an insertion head, in particular for an infusion set
Dispositif d'insertion pour une tête d'insertion, en particulier pour un ensemble d'infusion

(43) Veröffentlichungstag der Anmeldung: 17.09.2008
(73) Patentinhaber: F. Hoffmann-La Roche Ltd., 4070 Basel (CH); Roche Diagnostics GmbH, 68305 Mannheim (DE)
(72) Erfinder: Liniger, Jürg, 3072 Ostermundigen (CH); Rütti, Pascal, 4623 Neuendorf (CH); Pearson, Allen, Wisow Cambridgeshire PE28 2QB (GB); Kelsey, Tom, Cambridge CB5 8US (GB)
(74) Vertreter: Schalch, Rainer

(56) Entgegenhaltungen:
- WO-A-2004/101071
- WO-A-2005/065748
- US-A1- 2003 125 669
- US-A1- 2005 035 014

## Beschreibung

Die vorliegende Erfindung betrifft eine Insertionsvorrichtung für einen Insertionskopf sowie eine Anordnung umfassend die Insertionsvorrichtung und einen in dieser aufgenommenen oder aufnehmbaren Insertionskopf, gemäss den Oberbegriffen der unabhängigen Patentansprüche.

Bei Patienten, welche einen regelmässigen Bedarf an einem durch direkte Zuführung in das Körpergewebe oder in den Blutkreislauf verabreichbaren Medikament haben, wie z.B. bestimmte Gruppen von Schmerzpatienten oder Patienten mit Diabetes Typ I und Typ II, kann es sinnvoll sein, dem Körper die erforderliche Medikamentenmenge in flüssiger Form über eine an geeigneter Stelle in den Körper eingebrachte und über einen längeren Zeitraum dort verbleibende Kanüle zuzuführen. Hierzu wird auf der Haut des Patienten eine je nach Ausführung als "Infusionsset" oder "Port" bezeichnete Anordnung mit einer Kanüle befestigt, derart, dass die Kanüle durch die Haut in den Körper eindringt.

Auch gibt es zunehmend Bestrebungen, bestimmte medizinische Parameter eines Patienten, wie z.B. den Blutzuckerwert, über einen längeren Zeitraum kontinuierlich zu überwachen. Hierzu wird beispielsweise am Körper des Patienten eine Sensoranordnung angeordnet, welche mit einer Einstechspitze eines geeigneten Sensors durch die Haut in den Körper des Patienten eindringt.

Um Infektionen zu vermeiden, muss das Infusionsset, der Port oder die Sensoranordnung in regelmässigen Zeitintervallen gewechselt werden, z.B. alle drei Tage, was bei der nicht stationären Behandlung, z.B. von Diabetikern, oftmals durch den Patienten selbst erfolgt und infolge des Einstechens der Infusionskanüle oder der Einstechspitze mit gewissen Schmerzen verbunden ist. Entsprechend wichtig ist es, dass solche Infusionssets, Ports oder Sensoranordnungen einfach und sicher zu applizieren sind, weshalb viele Hersteller inzwischen dazu übergegangen sind, ihre Produkte als Insertionsköpfe für spezielle Insertionsvorrichtungen auszubilden, mit Hilfe welcher diese am Körper des Patienten appliziert werden können. Hierdurch wird das Applizieren erleichtert und der Applikationsschmerz infolge des schnellen und gezielten Einstechens auf ein Minimum reduziert.

So sind z.B. aus US 6,607,509 B2 Insertionsvorrichtungen für Infusionssets bekannt, bei denen das Infusionsset durch die Kraft einer vorgespannten Feder schlagartig auf die Applikationsstelle aufgesetzt wird, unter einem Einstechen der Kanüle in das Gewebe des Patienten. Nach erfolgter Applikation des Infusionssets muss die Insertionsvorrichtung vom Infusionsset abgekoppelt und entfernt werden, was den Nachteil birgt, dass es hierbei zu einer Irritation der Einstechstelle durch Kraftausübung auf die eingestochene Kanüle kommen kann.

WO 2004/110527 A1 offenbart neben Insertionsvorrichtungen wie zuvor beschrieben eine ähnliche Insertionsvorrichtung für Infusionssets, bei der das Infusionsset jedoch bereits zu Beginn der Insertionsbewegung automatisch von der Insertionsvorrichtung getrennt wird und sodann im Freiflug in den Körper des Patienten eindringt. Hierdurch ergibt sich gegenüber den zuvor beschriebenen Insertionsvorrichtungen der Vorteil, dass bei der eigentlichen Einstechbewegung praktisch keine Reibungsverluste innerhalb der Insertionsvorrichtung auftreten, so dass die Applikation mit hoher Geschwindigkeit und mit einer entsprechend kurzen Schmerzphase erfolgen kann. Auch wird eine Irritation der Einstechstelle durch ein nachträgliches Lösen der Insertionsvorrichtung vom Insertionskopf vermieden. Diese Insertionsvorrichtung weist jedoch den Nachteil auf, dass sie relativ kompliziert in der Anwendung ist und eine Vielzahl von Bedienungsschritten erfordert.

Es stellt sich daher die Aufgabe, eine Insertionsvorrichtung, eine Anordnung umfassend eine Insertionsvorrichtung mit einem zugehörigen Insertionskopf sowie ein Verfahren zum Applizieren eines Insertionskopfes zur Verfügung zu stellen, welche die Nachteile des Standes der Technik nicht aufweisen oder diese zumindest teilweise vermeiden.

Diese Aufgabe wird durch die Insertionsvorrichtung gemäss Patentanspruch 1 und die Anordnung gemäss Patentanspruch 22 gelöst.

Demgemäss betrifft ein erster Aspekt der Erfindung eine Insertionsvorrichtung für einen Insertionskopf mit mindestens einer Infusionskanüle und/oder mindestens einer Einstechspitze. Ein mit der Insertionsvorrichtung zu applizierender Insertionskopf kann also z.B. eine einzelne Infusionskanüle oder eine einzelne.Einstechspitze aufweisen, mehrere Kanülen oder mehrere Einstechspitzen aufweisen oder auch eine oder mehrere Kanülen und eine oder mehrere Einstechspitzen aufweisen und des Weiteren z.B. als Infusionsset, als Port und/oder als Sensoranordnung, z.B. zur Messung des Blutzuckerwertes, ausgebildet sein. Die Insertionsvorrichtung weist eine oder mehrere, beispielsweise von einem Gehäuse gebildete Kontaktflächen zum Aufsetzen derselben auf die Haut des Patienten beim Applizieren des Insertionskopfes auf. Weiter umfasst die Insertionsvorrichtung Haltemittel, z.B. zwei sich gegenüberliegende Blattfederelemente, mit denen der zu applizierende Insertionskopf vorübergehend an der Insertionsvorrichtung gehalten werden kann, so dass beim eigentlichen Applizieren des Insertionskopfes lediglich die Insertionsvorrichtung vom Bediener an der Applikationsstelle gehalten werden muss. Auch verfügt die Insertionsvorrichtung über Antriebsmittel, welche mindestens ein vorspannbares Kraftspeicherelement, z.B. eine Spiralfeder, zur Bereitstellung der Antriebsenergie aufweisen, mit denen der zu applizierende Insertionskopf bei applikationsbereiter Insertionsvorrichtung von einer ersten Position, in welcher er derartig mit den Haltemitteln gehalten ist, dass sämtliche seiner Infusionskanülen und Einstechspitzen zur Vermeidung eines unbeabsichtigten Inkontaktkommens mit dem Bediener gegenüber der Kontaktfläche zurückstehen, relativ zu der Kontaktfläche in Längsrichtung einer Infusionskanüle oder Einstechspitze in eine zweite Position, in welcher sämtliche seiner Infusionskanülen und Einstechspitzen im wesentlichen vollständig über die Kontaktfläche hinaus stehen, bewegt werden kann, zur Ermöglichung eines Einstechens dieser Infusionskanülen und Einstechspitzen in den Körper des Patienten bei mit der Kontaktfläche auf der Haut des Patienten aufgesetzter Insertionsvorrichtung. Diese Bewegung des Insertionskopfes, welche angetrieben durch das Kraftspeicherelement und unter zunehmender Entspannung desselben erfolgt und mit welcher das eigentliche Applizieren des Insertionskopfes am Körper bewerkstelligt wird, wird anspruchsgemäss als Insertionsbewegung bezeichnet. Die Haltemittel der Insertionsvorrichtung sind relativ zur Kontaktfläche in einer Einsetzposition und in einer Bereitschaftsposition positionierbar und derartig ausgestaltet, dass der zu applizierende Insertionskopf in der Einsetzposition an den Haltemitteln angeordnet werden kann, derart, dass er an diesen gehalten wird, und die Haltemittel sodann ausgehend von der Einsetzposition mit dem daran gehaltenen Insertionskopf in die Bereitschaftsposition bringbar sind. Dabei wird der Insertionskopf bei Erreichen der Bereitschaftsposition von den Haltemitteln applikationsbereit, d.h. in einstechbereitem Zustand, in der ersten Position gehalten. Zudem ist die Insertionsvorrichtung derartig ausgebildet, dass der Insertionskopf zu Beginn der Insertionsbewegung von den Haltemitteln separiert wird, so dass er den grössten Teil der Insertionsbewegung losgelöst von den Haltemitteln vollführen kann, bevorzugterweise im "Freiflug" oder allenfalls durch seitliche Führungen geführt, und nach dem Applizieren keine Verbindung mehr zwischen dem Insertionskopf und der Insertionsvorrichtung vorliegt, so dass die Insertionsvorrichtung von dem am Körper applizierten Insertionskopf entfernt werden kann, ohne die Gefahr einer Irritation der Applikationsstelle. Weiter ist die Insertionsvorrichtung derartig ausgebildet, dass das vorspannbare Kraftspeicherelement beim Bewegen der Haltemittel mit dem daran gehaltenen Insertionskopf von der Einsetzposition in die Bereitschaftsposition automatisch vorgespannt wird.

Durch die Erfindung wird die Bereitstellung von Insertionsvorrichtungen für Insertionsköpfe ermöglicht, welche eine sehr kurze Einstechphase mit einem entsprechend kurzen Einstechschmerz ermöglichen, eine Irritation der Einstichstelle beim Lösen des Insertionskopfes von der Insertionsvorrichtung verhindern und gleichzeitig wesentlich einfacher in der Handhabung sind als die heute bekannten gattungsgemässen Insertionsvorrichtungen.

In einer bevorzugten Ausführungsform ist die Insertionsvorrichtung derartig ausgebildet, dass der Insertionskopf in der ersten Position rein kraftschlüssig, rein formschlüssig oder kraft- und formschlüssig von den Haltemitteln gehalten werden kann. Insbesondere bei reinem Kraftschluss ergibt sich der Vorteil, dass der Insertionskopf zu Beginn der Insertionsbewegung auf einfache Weise durch ein Vortriebselement der Antriebsmittel aus den Haltemitteln geschlagen werden kann und so von diesen gelöst wird.

Dabei ist es weiter bevorzugt, dass die Insertionsvorrichtung derartig ausgebildet ist, dass der Insertionskopf in der Einsetzposition rein formschlüssig oder kraft- und formschlüssig von den Haltemitteln gehalten werden kann, wobei es bevorzugt ist, dass die Insertionsvorrichtung derartig ausgebildet ist, das der Formschluss beim Bewegen der Haltemittel mit dem darin gehaltenen Insertionskopf von der Einsetzposition in die Bereitschaftsposition aufgehoben wird.

Alternativ ist es dabei weiter bevorzugt, dass die Insertionsvorrichtung derartig ausgebildet ist, dass der Insertionskopf in der Einsetzposition rein kraftschlüssig von den Haltemitteln gehalten werden kann, wobei es bevorzugt ist, dass die Insertionsvorrichtung derartig ausgebildet ist, dass der Kraftschluss beim Bewegen der Haltemittel mit dem darin gehaltenen Insertionskopf von der Einsetzposition in die Bereitschaftsposition verringert wird. Dies kann beispielsweise dadurch erreicht werden, dass der Insertionskopf kraftschlüssig mit Federlaschen gehalten wird, deren freie federnde Länge beim Bewegen der Haltemittel von der Einsetzposition in die Bereitschaftsposition verlängert wird. Ebenso sind aber auch Ausführungsvarianten vorgesehen, bei denen sowohl in der Einsetzposition als auch in der Bereitschaftsposition ein reiner Kraftschluss gleicher Stärke vorliegt.

Insbesondere bei der Verwendung von Insertionsköpfen mit feststehender Infusionskanüle oder Einstechspitze ergibt sich bei den Ausführungsvarianten, bei denen bei der Bewegung der Haltemittel mit dem darin gehaltenen Insertionskopf von der Einsetzposition in die Bereitschaftsposition ein Formschluss aufgehoben oder ein Kraftschluss verringert wird, der Vorteil, dass der Insertionskopf in der Einsetzposition fester gehalten wird als in der Bereitschaftsposition, so dass ein Entfernen des Nadel- bzw. Kanülenschutzes möglich ist ohne Gefahr zu laufen, dabei den Insertionskopf wieder aus den Haltemitteln zu lösen.

In noch einer bevorzugten Ausführungsform ist die Insertionsvorrichtung derartig ausgebildet, dass die Haltemittel zumindest während eines Grossteils der Insertionsbewegung oder während der gesamten Insertionsbewegung des Insertionskopfes unbewegt gegenüber der Kontaktfläche bzw. den Kontaktflächen sind. Auf diese Weise lassen sich konstruktiv einfach aufgebaute erfindungsgemässe Insertionsvorrichtungen bereitstellen.

In einer weiteren bevorzugten Ausführungsform weist die Insertionsvorrichtung manuell betätigbare Aktivierungsmittel wie z.B. ein Schieberelement, einen Drehknopf oder einen Drucktaster auf, mittels welcher die Haltemittel mit dem daran gehaltenen Insertionskopf manuell durch Muskelkraft, also durch Manipulation mittels einer Hand oder beiden Händen, von der Einsetzposition in die Bereitschaftsposition gebracht werden können, unter gleichzeitigem Vorspannen des Kraftspeicherelements.

Dabei ist es bevorzugt, wenn die Insertionsvorrichtung ein Gehäuse umfasst und die Haltemittel bevorzugterweise fest mit einem als Schieberelement ausgebildeten Aktivierungsmittel verbunden sind, welches von Hand ergreifbar und gegenüber dem Gehäuse bewegbar, bevorzugterweise verschiebbar ist, zum Bewegen der Haltemittel von der Einsetzposition in die Bereitschaftsposition unter gleichzeitigem Vorspannen des Kraftspeicherelements.

Hierdurch wird die Bereitstellung von kostengünstigen, rein mechanischen Insertionsvorrichtungen ermöglicht, welche jederzeit ohne Hilfsenergie einsatzbereit sind. Dies im Gegensatz zu ebenfalls vorgesehenen bevorzugten Ausführungsformen der erfindungsgemässen Insertionsvorrichtung, bei denen das Vorspannen mittels elektrischer Elemente, wie z.B. mittels eines Elektromotors, erfolgt. Bei solchen Ausführungsformen können die Aktivierungsmittel Schalter und/oder Sensoren, welche die elektrischen Elemente zum Vorspannen der Kraftspeicherelemente ansteuern, umfassen.

In noch einer weiteren bevorzugten Ausführungsform ist die Insertionsvorrichtung derartig ausgebildet, dass ihre Gesamtabmessungen beim Bewegen der Haltemittel von der Einsetzposition in die Bereitschaftsposition unverändert bleiben. Solche Vorrichtungen weisen den Vorteil auf, dass sie besonders kompakt und robust ausgebildet werden können und im Lagerzustand, in welchem die Kraftspeicherelemente entspannt bzw. nicht applikationsbereit vorgespannt sein sollten, um ein Nachlassen der Spannkraft zu vermeiden, keine grösseren Abmessungen aufweisen als im vorgespannten, applikationsbereiten Zustand, was die Lagerung bzw. den Transport der Applikationsvorrichtung, z.B. in einer Handtasche, erleichtert.

Auch ist es von Vorteil, wenn die Insertionsvorrichtung derartig ausgestaltet ist, dass das Kraftspeicherelement wiederholt durch den Bediener vorspannbar ist, so dass die Insertionsvorrichtung mehrfach zum Applizieren eines Insertionskopfes verwendet werden kann.

Weiter ist es bevorzugt, dass die Insertionsvorrichtung derartig ausgestaltet ist, dass das Kraftspeicherelement bei in der ersten Position befindlichem Insertionskopf im vorgespannten Zustand bereitstellbar ist und sodann die Insertionsbewegung durch Betätigen eines Betätigungsorgans oder durch ein gleichzeitiges oder aufeinanderfolgendes Betätigen mehrerer Betätigungsorgane auslösbar ist, unter Entspannung des Kraftspeicherelements. Geeignete Betätigungsorgane können rein mechanisch ausgebildet sein, z.B. als Auslöseriegel oder Auslöseschieber, oder z.B. auch elektrische bzw. elektronische Elemente umfassen, z.B. einen elektrisch betätigten Riegel, welcher über einen Schalter und/oder einen Sensor mit Auswerteelektronik gelöst werden kann. Hierdurch wird eine kontrollierte Applikation des Insertionskopfes begünstigt.

Dabei ist es zudem vorteilhaft, wenn die Antriebsmittel ein Vortriebselement zur Übertragung der Antriebskraft auf den zu applizierenden Insertionskopf umfassen und derartig ausgestaltet sind, dass das Kraftspeicherelement unter einem Verschieben des Vortriebselements entgegen der Richtung der Insertionsbewegung und unter anschliessendem Verrasten desselben mit durch die Betätigungsorgane lösbaren Rastmitteln vorspannbar und in vorgespanntem Zustand bereitstellbar ist. Solche Konstruktionen lassen sich kostengüstig realisieren, sind funktionssicher und ermöglichen zudem eine hohe Anfangsbeschleunigung und damit eine schnelle Insertionsbewegung, wodurch sich der Applikationsschmerz beim Einstechen der Infusionskanüle oder der Einstechspitze in den Körper minimieren lässt.

Auch ist es bei erfindungsgemässen Insertionsvorrichtungen, bei denen das Kraftspeicherelement bei in der ersten Position befindlichem Insertionskopf im vorgespannten Zustand bereitstellbar ist und sodann die Insertionsbewegung durch Betätigen eines Betätigungsorgans auslösbar ist, bevorzugt, dass die Insertionsvorrichtung mindestens zwei Betätigungsorgane aufweist, welche zur Auslösung der Insertionsbewegung gleichzeitig betätigt sein müssen. Dabei ist ein erstes der Betätigungsorgane bevorzugterweise derartig ausgebildet, dass es durch ein Andrücken der Insertionsvorrichtung mit der Kontaktfläche an den Körper des Patienten betätigt werden kann, und zwar bevorzugterweise durch ein Andrücken derselben in Richtung der Insertionsbewegung, was insbesondere dann sinnvoll ist, wenn die Insertion unter einem Winkel von etwa 90° zur Körperoberfläche erfolgt. Durch diese Ausgestaltung der Insertionsvorrichtung wird die Gefahr eines versehentlichen Auslösens der applikationsbereiten Insertionsvorrichtung und damit die Wahrscheinlichkeit einer Verletzung des Bedieners deutlich herabgesetzt.

Bevorzugterweise ist dabei das erste Betätigungsorgan als schieber- oder tasterförmiges Element ausgebildet , wobei es weiter bevorzugt ist, wenn dieses gleichzeitig die gesamte Kontaktfläche oder aber, bei mehreren Kontaktflächen, sämtliche Kontaktflächen der Insertionsvorrichtung bildet. Hierdurch ergibt sich der Vorteil, dass eine sichere Betätigung des ersten Betätigungsorgans unabhängig von der Oberflächenkontur der Applikationsstelle am Körper des Patienten möglich ist.

Alternativ zu rein mechanischen Ausführungsformen des ersten Betätigungsorgans ist es auch bevorzugt, elektrische erste Betätigungsorgane vorzusehen. Dies ist beispielsweise möglich in Form eines elektrischen Riegelelements, welches mittels eines oder mehrerer an der Kontaktfläche angeordneter Hautkontaktsensoren (z.B. Leitfähigkeitssensoren) und einer zugeordneten Steuerelektronik angesteuert wird, derart, dass dieses beim bestimmungsgemässen Aufsetzen der Insertionsvorrichtung auf die Applikationsstelle aktiviert wird.

Zudem ist es bei Ausführungsformen der Insertionsvorrichtung mit mindestens zwei Betätigungsorganen bevorzugt, dass zumindest eines der Betätigungsorgane, anspruchsgemäss als zweites Betätigungsorgan bezeichnet, als tasten- oder knopfförmiges Element ausgebildet ist, welches durch Drücken mit einer Fingerkuppe des Bedieners betätigt werden kann. Dabei ist die Betätigungsrichtung bevorzugterweise quer, insbesondere senkrecht zur Andrückrichtung der Insertionsvorrichtung an den Körper des Patienten, welche bevorzugterweise gleich der Richtung der anspruchsgemässen Insertionsbewegung und damit der Einstichrichtung der Infusionskanüle oder der Einstechspitze ist. Dieses zweite Betätigungsorgan kann rein mechanisch ausgebildet sein, z.B. als Auslöseriegel oder Auslöseschieber, oder z.B. auch elektrische bzw. elektronische Elemente umfassen, z.B. einen elektrisch betätigten Riegel, welcher über einen Schalter und/oder einen Sensor mit Auswerteelektronik gelöst werden kann.

Durch diese Ausgestaltung lässt sich die Gefahr einer unbeabsichtigten Betätigung dieses Betätigungsorgans zusammen mit dem anspruchsgemässen ersten Betätigungsorgan weiter herabsetzten, so dass die Gefahr einer unbeabsichtigten Auslösung der Insertionsvorrichtung weiter gesenkt wird.

Bei bestimmten Ausführungsformen kann es auch vorteilhaft sein, wenn die Betätigungsrichtung parallel oder im wesentlichen parallel zur Andrückrichtung ist.

In noch einer weiteren bevorzugten Ausführungsform der erfindungsgemässen Insertionsvorrichtung mit mindestens zwei Betätigungsorganen sind deren für die Auslösung der Insertionsbewegung zu betätigenden Betätigungsorgane derartig miteinander gekoppelt, dass durch die Betätigung eines dieser Betätigungsorgane eine Sperrung eines anderen oder mehrerer anderer dieser Betätigungsorgane aufhebbar ist. Hierdurch kann die Konstruktion vereinfacht werden, da nur ein einziger Auslösemechanismus erforderlich ist. Insbesondere bei Vorhandensein eines Betätigungsorgans mit elektrischen Elementen kann hierdurch ein besonders einfacher kostengünstiger Aufbau realisiert werden, z.B. indem ein elektrisches Riegelelement nur dann auslösbar ist, wenn zwei Schalter oder Sensoren gleichzeitig betätigt werden oder eine Kombination aus mindestens einem Schalter und mindestens einem Sensor.

In noch einer weiteren bevorzugten Ausführungsform der Insertionsvorrichtung mit einem oder mindestens zwei Betätigungsorganen sind sämtliche Betätigungsorgane, welche zur Auslösung der Insertionsbewegung gleichzeitig betätigt sein müssen, derartig ausgebildet, dass sie vom Bediener mit einer Hand betätigt werden können. Entsprechend ist eine einhändige Bedienung der Insertionsvorrichtung möglich, wodurch die Applikation des Insertionskopfes durch den Patienten selbst an für diesen beidhändig nicht oder nur sehr schwer zugänglichen Körperstellen, wie z.B. im Bereich der Hüfte, möglich wird.

In einer bevorzugten Ausführungsform der Insertionsvorrichtung mit einem oder mehreren Betätigungsorganen sind die Betätigungsorgane, welche zur Auslösung der Insertionsbewegung gleichzeitig betätigt sein müssen, derartig ausgebildet, dass diese bei Wegfall einer Betätigungskraft automatisch wieder ihren unbetätigten Zustand annehmen. Hierdurch lässt sich die Sicherheit gegen ein unbeabsichtigte Auslösen der Insertionsvorrichtung weiter steigern.

Weisen die Antriebsmittel der Insertionsvorrichtung ein oder mehrere Kraftspeicherelemente zur Bereitstellung der Antriebsenergie für die Insertionsbewegung auf, wie z.B. Spiral-, Schenkel- oder Blattfedern aus Metall oder Kunststoff, Gasdruckfedern oder Gummifederelemente, was bevorzugt ist, so ist die Insertionsvorrichtung unabhängig von etwaigen Fremdenergiequellen jederzeit und überall einsatzfähig und kann zudem besonders kostengünstig ausgebildet werden.

In noch einer bevorzugten Ausführungsform weist die Insertionsvorrichtung Mittel zur Bewirkung einer Verschiebung, insbesondere einer Querverschiebung, eines verschieblichen Aktivierungsorgans eines in den Haltemitteln aufzunehmenden Insertionskopfes mit einer einzigen oder mehreren ausklappbaren Infusionskanülen und/oder einer einzigen oder mehreren ausklappbaren Einstechspitzen während dem Bewegen der Haltemittel mit dem darin bestimmungsgemäss gehaltenen Insertionskopf von der Einsetzposition in die Bereitschaftsposition auf, zur Ermöglichung eines automatischen Ausklappens sämtlicher ausklappbarer Infusionskanülen und sämtlicher ausklappbarer Einstechspitzen des Insertionskopfes während dem Bewegen von der Einsetzposition in die Bereitschaftsposition. Hierdurch wird es möglich, auf die Insertionsvorrichtung angepasste Insertionsköpfe mit ausklappbaren Infusionskanülen bzw. Einstechspitzen im Schutzzustand, d.h. mit eingeklappten Kanülen bzw. Einstechspitzen, in die in der Einsetzposition positionierten Haltemittel der Insertionsvorrichtung einzusetzen und den Insertionskopf sodann automatisch durch Bewegen desselben mit den Haltemitteln in die erste Position unter gleichzeitigem Vorspannen des Kraftspeicherelements und Ausklappen der Kanülen bzw. Einstechspitzen applikationsbereit zu machen. Hierdurch kann die Gefahr einer Verletzung des Bedieners mit den Kanülen oder den Einstechspitzen bei der Vorbereitung der Applikation des Insertionskopfes praktisch eliminiert werden.

Bei der zuvor beschriebenen Ausführungsform ist es bevorzugt, dass die Mittel zur Bewirkung einer Verschiebung eines verschieblichen Aktivierungsorgans eine Rampenfläche umfassen bzw. als Rampenfläche ausgebildet sind, an welcher ein querverschiebliches Aktivierungsorgan eines entsprechend ausgebildeten Insertionskopfes während dem Bewegen der Haltemittel mit dem darin bestimmungsgemäss gehaltenen Insertionskopf von der Einsetzposition in die Bereitschaftsposition anlaufen und dabei quer zur Bewegungsrichtung der Haltemittel und des Insertionskopfes verschoben werden kann, zur Bewirkung eines Ausklappens der ausklappbaren Infusionskanülen bzw. Einstechspitzen des Insertionskopfes. Solche Mittel zur Bewirkung einer Verschiebung eines verschieblichen Aktivierungsorgans lassen sich denkbar einfach und kostengünstig zur Verfügung stellen.

Bei den beiden zuvor beschriebenen Ausführungsformen ist es auch bevorzugt, dass die Mittel zur Bewirkung einer Verschiebung eines verschieblichen Aktivierungsorgans einen Hebelmechanismus umfassen, mit welchem ein verschiebliches, insbesondere querverschiebliches Aktivierungsorgan eines entsprechend ausgebildeten Insertionskopfes während dem Bewegen der Haltemittel mit dem darin bestimmungsgemäss gehaltenen Insertionskopf von der Einsetzposition in die Bereitschaftsposition insbesondere quer zur Bewegungsrichtung der Haltemittel und des Insertionskopfes verschoben werden kann, zur Bewirkung eines Ausklappens der ausklappbaren Infusionskanülen bzw. Einstechspitzen des Insertionskopfes. Hierdurch ergeben sich grosse Freiräume für die konstruktive Ausgestaltung sowohl der Insertionsvorrichtung als auch des zugehörigen Insertionskopfes.

Ein zweiter Aspekt der Erfindung betrifft eine Anordnung, welche eine Insertionsvorrichtung gemäss dem ersten Aspekt der Erfindung und einen in dieser aufgenommenen oder aufnehmbaren, bevorzugterweise als Infusionsset, Port und/oder Sensoranordnung ausgebildeten Insertionskopf mit mindestens einer Infusionskanüle und/oder mindestens einer Einstechspitze umfasst. Der Insertionskopf kann also z.B. eine einzelne Infusionskanüle oder Einstechspitze aufweisen, mehrere Kanülen oder Einstechspitzen aufweisen oder auch eine oder mehrere Kanülen und eine oder mehrere Einstechspitzen umfassen. Durch Einsetzen eines entsprechenden passenden, zu applizierenden Insertionskopfes in die erfindungsgemässe Insertionsvorrichtung gemäss dem ersten Aspekt der Erfindung entsteht zwangsläufig eine solche erfindungsgemässe Anordnung. Dabei ist es neben der Vermarktung von mehrfach verwendbaren erfindungsgemässen Insertionsvorrichtungen gemäss dem ersten Aspekt der Erfindung und zugehörigen Insertionsköpfen, wie zugehörigen Infusionssets, Ports oder Sensoranordnungen, welche vom Bediener kurz vor der Verwendung zu erfindungsgemässen Anordnungen zusammengefügt werden, auch vorgesehen, bereits fertig zusammengestellte erfindungsgemässe Anordnungen als Einwegartikel anzubieten, bei denen die Insertionsvorrichtung nach dem Applizieren des Insertionskopfes entsorgt wird.

In einer bevorzugten Ausführungsform der erfindungsgemässen Anordnung ist der Insertionskopf ein Insertionskopf mit mindestens einer ausklappbaren Infusionskanüle und/oder mindestens einer ausklappbaren Einstechspitze, wobei sämtliche ausklappbaren Infusionskanülen und Einstechspitzen des Insertionskopfes im nicht-aktivierten Zustand, in welchem der Insertionskopf zum Einsetzen in die in der Einsetzposition angeordneten Haltemittel vorgesehen ist oder bereits in den in der Einsetzposition angeordneten Haltemitteln gehalten wird, eingeklappt sind und die Insertionsvorrichtung und der Insertionskopf derartig ausgebildet sind, dass sämtliche ausklappbaren Infusionskanülen und Einstechspitzen des Insertionskopfes beim Bewegen der Haltemittel mit dem darin bestimmungsgemäss gehaltenen Insertionskopf von der Einsetzposition in die Bereitschaftsposition ausgeklappt werden. Wie bereits anlässlich der Diskussion des ersten Aspekts der Erfindung erwähnt, kann hierdurch die Gefahr einer Verletzung des Bedieners an einer Kanüle oder Einstechspitze bei der Vorbereitung der Applikation des Insertionskopfes praktisch ausgeschlossen werden.

Bei Verwendung der Insertionsvorrichtung oder der Anordnung nach einem der vorangehenden Aspekte der Erfindung zum Applizieren eines bevorzugterweise als Infusionsset, Port oder Sensoranordnung ausgebildeten Insertionskopfes mit mindestens einer Infusionskanüle und/oder mindestens einer Einstechspitze am Körper eines Patienten treten die Vorteile der Erfindung besonders deutlich zu Tage.

Bei einem Verfahren zum Applizieren eines bevorzugterweise als Infusionsset, Port oder Sensoranordnung ausgebildeten Insertionskopfes am Körper eines Patienten mit einer Insertionsvorrichtung gemäss dem ersten Aspekt der Erfindung wird in einem ersten Verfahrensschritt die Insertionsvorrichtung mit den Haltemitteln angeordnet in der Einsetzposition bereitgestellt.

Sodann wird in einem zweiten Schritt ein auf die Insertionsvorrichtung angepasster Insertionskopf mit mindestens einer Infusionskanüle und/oder mindestens einer Einstechspitze in die Haltemittel eingesetzt, derart, dass dieser von den Haltemitteln gehalten wird. Es liegt also eine erfindungsgemässe Anordnung gemäss dem zweiten Aspekt vor, wobei der Insertionskopf eine einzelne Infusionskanüle oder Einstechspitze, mehrere Kanülen oder Einstechspitzen oder auch eine oder mehrere Kanülen und eine oder mehrere Einstechspitzen aufweisen kann.

Sodann werden in einem dritten Schritt die Haltemittel ausgehend von der Einsetzposition mit dem bestimmungsgemäss daran gehaltenen Insertionskopf unter einem Vorspannen des Kraftspeicherelements der Insertionsvorrichtung in die Bereitschaftsposition bewegt, wobei der Insertionskopf nach dem Erreichen der Bereitschaftsposition durch die Haltemittel applikationsbereit in der ersten Position gehalten wird. Die Insertionsvorrichtung ist nun bereit für den eigentlichen Applikationsvorgang.

Hierzu wird die Insertionsvorrichtung in einem vierten Schritt des Verfahrens mit der Kontaktfläche der Insertionsvorrichtung an der gewünschten Applikationsstelle am Körper des Patienten derartig angeordnet, dass die Infusionskanülen bzw. Einstechspitzen des Insertionskopfes bei der anschliessend zu erfolgenden Insertionsbewegung bestimmungsgemäss in den Körper eindringen können.

Sodann wird in einem fünften Schritt die Insertionsbewegung des Insertionskopfes ausgelöst, wobei der Insertionskopf zu Beginn der Insertionsbewegung von den Haltemitteln separiert wird, derart, dass dieser zumindest den grössten Teil der Insertionsbewegung losgelöst von den Haltemitteln vollführt.

Durch dieses Verfahren zum Applizieren eines Insertionskopfes mit einer Insertionsvorrichtung am Körper eines Patienten wird eine kurze Einstechphase mit einem entsprechend kurzen Einstechschmerz ermöglicht und eine Irritation der Einstichstelle beim Lösen des Insertionskopfes von der Insertionsvorrichtung kann sicher verhindert werden, bei gleichzeitiger Vereinfachung der Verfahrensdurchführung gegenüber heute bekannten Verfahren.

In einer bevorzugten Ausführungsform des Verfahrens wird der Insertionskopf in der ersten Position rein kraftschlüssig, rein formschlüssig oder kraft- und formschlüssig mit den Haltemitteln gehalten. Hierdurch ergibt sich insbesondere bei reinem Kraftschluss der Vorteil, dass der Insertionskopf zu Beginn der Insertionsbewegung auf einfache Weise durch ein Vortriebselement der Antriebsmittel aus den Haltemittel geschlagen werden kann und so von diesen gelöst wird.

Dabei ist es bevorzugt, dass der Insertionskopf nach dem Einsetzen in die Haltemittel in der Einsetzposition rein formschlüssig oder kraft- und formschlüssig mit den Haltemitteln gehalten wird, wobei es bevorzugt ist, dass der Formschluss beim Bewegen der Haltemittel mit dem darin gehaltenen Insertionskopf von der Einsetzposition in die Bereitschaftsposition aufgehoben wird.

Alternativ ist es dabei bevorzugt, dass der Insertionskopf nach dem Einsetzen in die Haltemittel in der Einsetzposition rein kraftschlüssig mit den Haltemitteln gehalten wird, wobei es bevorzugt ist, dass der Kraftschluss beim Bewegen der Haltemittel mit dem darin gehaltenen Insertionskopf von der Einsetzposition in die Bereitschaftsposition verringert wird. Es sind aber auch Ausführungsformen vorgesehen, bei denen der Insertionskopf sowohl in der Einsetzposition als auch in der Bereitschaftsposition rein kraftschlüssig mit einem jeweils gleich starken Kraftschluss gehalten wird.

Insbesondere bei der Verwendung von Insertionsköpfen mit feststehenden Infusionskanülen bzw. Einstechspitzen ergibt sich bei denjenigen Verfahrensvarianten, bei denen bei der Bewegung der Haltemittel mit dem darin gehaltenen Insertionskopf von der Einsetzposition in die Bereitschaftsposition ein Formschluss aufgehoben oder ein Kraftschluss verringert wird, der Vorteil, dass der Insertionskopf in der Einsetzposition fester gehalten wird als in der Bereitschaftsposition, so dass ein Entfernen des Nadel- bzw. Kanülenschutzes möglich ist ohne Gefahr zu laufen, dabei der Insertionskopf wieder aus den Haltemitteln zu lösen.

In einer weiteren bevorzugten Ausführungsform des Verfahrens werden die Haltemittel zumindest während eines Grossteils der Insertionsbewegung oder während der gesamten Insertionsbewegung des Insertionskopfes unbewegt gegenüber der Kontaktfläche gehalten. Hierdurch ergibt sich der Vorteil, dass kostengünstige, konstruktiv einfach aufgebaute erfindungsgemässe Insertionsvorrichtungen für die Durchführung des Verfahrens verwendet werden können.

In noch einer weiteren bevorzugten Ausführungsform des Verfahrens werden die Haltemittel mit dem daran gehaltenen Insertionskopf manuell durch Muskelkraft von der Einsetzposition in die Bereitschaftsposition gebracht, und zwar bevorzugterweise durch Verschieben eines Schieberelements, Verdrehen eines Drehknopfes oder Drücken eines Drucktasters. Derartige Manipulationen sind besonders geeignet zur Übertragung der für das Vorspannen des Kraftspeicherelements und das Verschieben der Haltemittel erforderlichen Kraft auf die Insertionsvorrichtung.

In noch einer weiteren bevorzugten Ausführungsform des Verfahrens wird das Kraftspeicherelement im vorgespannten Zustand bereitgestellt und sodann die Insertionsbewegung durch Betätigen eines Betätigungsorgans oder durch ein gleichzeitiges oder aufeinander folgendes Betätigen mehrerer Betätigungsorgane ausgelöst. Dies begünstigt einen kontrollierten Applikationsvorgang.

Auch ist es bevorzugt, dass das Anordnen der Insertionsvorrichtung am Körper des Patienten und das Auslösen der Insertionsbewegung mit einer Hand erfolgt, so dass auch schwierig und nur mit einer Hand erreichbare Körperstellen für die Applikation bereitstehen.

In noch einer weiteren bevorzugten Ausführungsform des Verfahrens wird ein Insertionskopf mit mindestens einer ausklappbaren Infusionskanüle und/oder mindestens einer ausklappbaren Einstechspitze in einem Zustand, in welchem sämtliche ausklappbaren Infusionskanülen und Einstechspitzen des Insertionskopfes eingeklappt sind, in die Haltemittel eingesetzt. Sodann werden sämtliche ausklappbaren Infusionskanülen und Einstechspitzen des Insertionskopfes beim Bewegen der Haltemittel mit dem darin bestimmungsgemäss gehaltenen Insertionskopf von der Einsetzposition in die Bereitschaftsposition ausgeklappt. Der Insertionskopf ist sodann applikationsbereit. Hierdurch lässt sich die Verletzungsgefahr für den Bediener bei der Vorbereitung der Applikation praktisch auf Null reduzieren.

Weitere bevorzugte Ausführungen der Erfindung ergeben sich aus den abhängigen Ansprüchen und aus der nun folgenden Beschreibung anhand der Figuren. Dabei zeigen:
Fig. 1 einen Vertikalschnitt durch eine erste Ausführungsform der erfindungsgemässen Insertionsvorrichtung im nicht vorgespannten Zustand ohne Insertionskopf;
Fig. 2 eine Darstellung wie Fig. 1 der Insertionsvorrichtung aus Fig. 1, jedoch mit darin angeordnetem Insertionskopf;
Fig. 3 einen Vertikalschnitt durch die Insertionsvorrichtung aus den Figuren 1 und 2 während dem Vorspannen mit dem darin angeordnetem Insertionskopf;
Fig. 4 einen Vertikalschnitt durch die Insertionsvorrichtung aus den vorherigen Figuren im vorgespannten Zustand mit darin angeordnetem Insertionskopf in gesichertem, applikationsbereiten Zustand;
Fig. 5 eine Darstellung wie Fig. 4 der Insertionsvorrichtung aus Fig. 4, jedoch in entsichertem Zustand;
Fig. 6 eine Darstellung wie Fig. 5 der Insertionsvorrichtung aus Fig. 5, jedoch kurz nach dem Betätigen der Auslösetaste;
Fig. 7 einen Vertikalschnitt durch eine zweite Ausführungsform der erfindungsgemässen Insertionsvorrichtung im nicht vorgespannten Zustand mit darin angeordnetem Insertionskopf;
Fig. 8 einen Vertikalschnitt durch die Insertionsvorrichtung aus Fig. 7 im vorgespannten, entsicherten Zustand;
Fig. 9 einen Vertikalschnitt durch eine dritte Ausführungsform der erfindungsgemässen Insertionsvorrichtung im nicht vorgespannten Zustand mit darin angeordnetem Insertionskopf; und
Fig. 10 einen Vertikalschnitt durch die Insertionsvorrichtung aus Fig. 9 im vorgespannten, entsicherten Zustand.

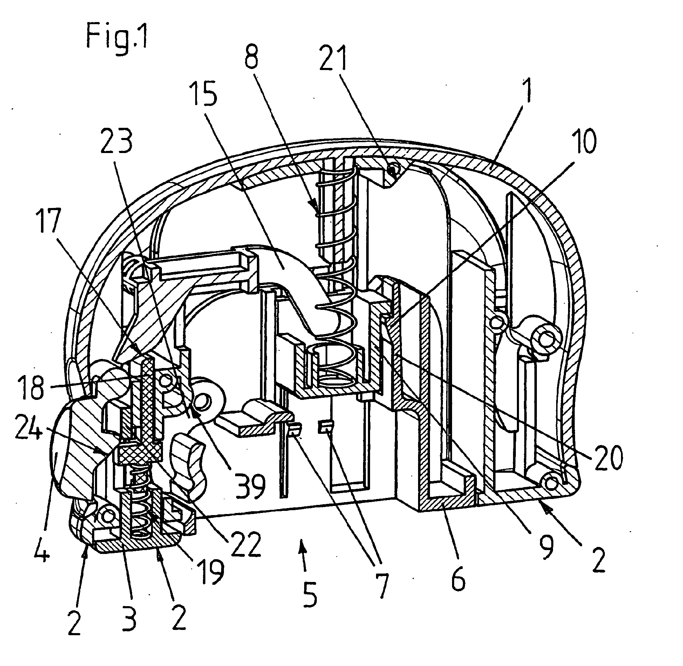

Eine erste Ausführungsform der erfindungsgemässen Insertionsvorrichtung für Insertionsköpfe mit ausklappbarer Infusionskanüle ist in einem Vertikalschnitt im nicht vorgespannten Zustand und ohne Insertionskopf in Fig. 1 dargestellt. Wie zu erkennen ist, weist die Insertionsvorrichtung ein portalartiges Gehäuse 1 auf, welches an seiner Unterseite Kontaktflächen 2 zum Aufsetzen und Andrücken der Insertionsvorrichtung auf den Körper eines Patienten beim Applizieren eines Infusionssets mit der Insertionsvorrichtung aufweist. Eine der Kontaktflächen 2 wird von einem Sicherungstaster 3 gebildet, welcher an der Unterseite des Gehäuses 1 nach unten vorsteht und zum Entsichern der Insertionsvorrichtung, wenn sich diese in einem applikationsbereiten Zustand befindet, durch Aufsetzen auf und Andrücken an den Körper des Patienten in eine Entsicherungsposition verschoben werden kann, in welcher die Kontaktfläche 2 des Sicherungstasters 3 im wesentlichen oberflächenbündig mit der an den Sicherungstaster 3 angrenzenden Kontaktfläche 2 des Gehäuses 1 ist.

Weiter weist die Insertionsvorrichtung einen Auslöseknopf 4 auf, mit welchem bei in einem applikationsbereiten Zustand sich befindlicher Insertionsvorrichtung und bei in der Entsicherungsposition angeordnetem Sicherungstaster 3 die Insertionsbewegung zum Applizieren des Insertionskopfes am Körper des Patienten ausgelöst werden kann. Sicherungstaster 3 und Auslöseknopf 4 stellen also zwei anspruchsgemässe Betätigungsorgane 3, 4 dar, welche zur Auslösung der Insertionsbewegung gleichzeitig betätigt sein müssen.

Wie ersichtlich ist, weist die Insertionsvorrichtung in der in Fig. 1 dargestellten Situation auf ihrer Unterseite eine Aufnahmeöffnung 5 auf, welche von einem Schieberelement 6 gebildet wird und in welcher vom Schieberelement 6 federelastisch gelagerte Vorsprünge 7 bereitgestellt werden, mit denen ein zu applizierendes Infusionsset, im vorliegenden Fall ausschliesslich kraftschlüssig, in der Aufnahmeöffnung 5 gehalten werden kann.

Wie weiter zu erkennen ist, umfasst die Insertionsvorrichtung als Antriebsmittel eine Spiralfeder 8, die auf ein Hammerelement 9 wirkt, welches sich in der dargestellten Situation auf einem Rastvorsprung 10 des Schieberelements 6 abstützt.

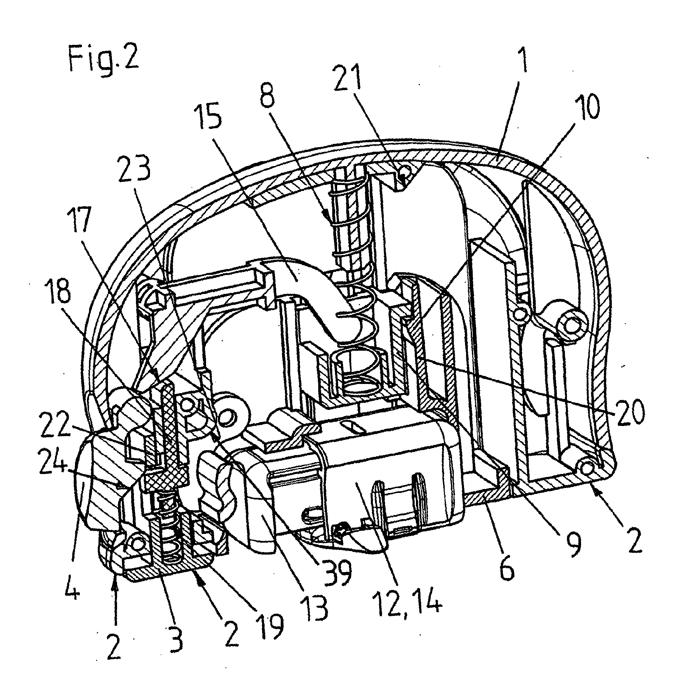

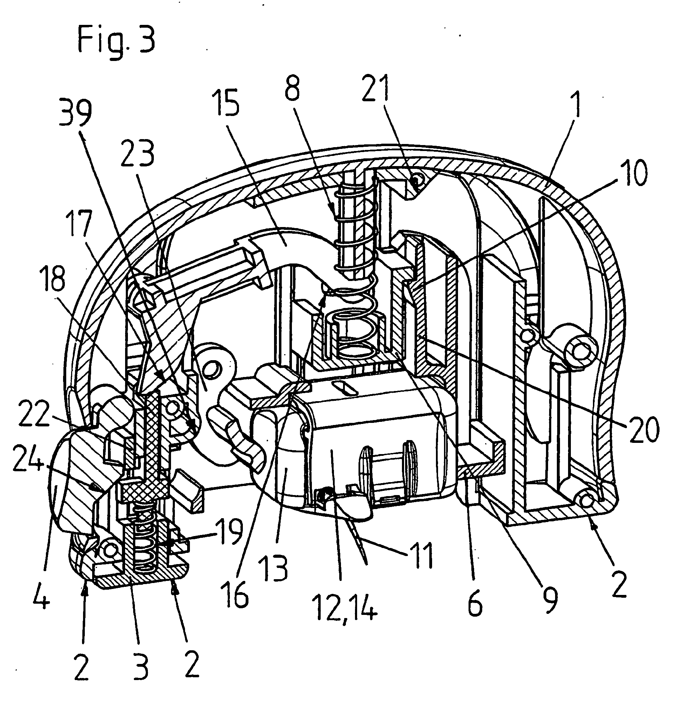

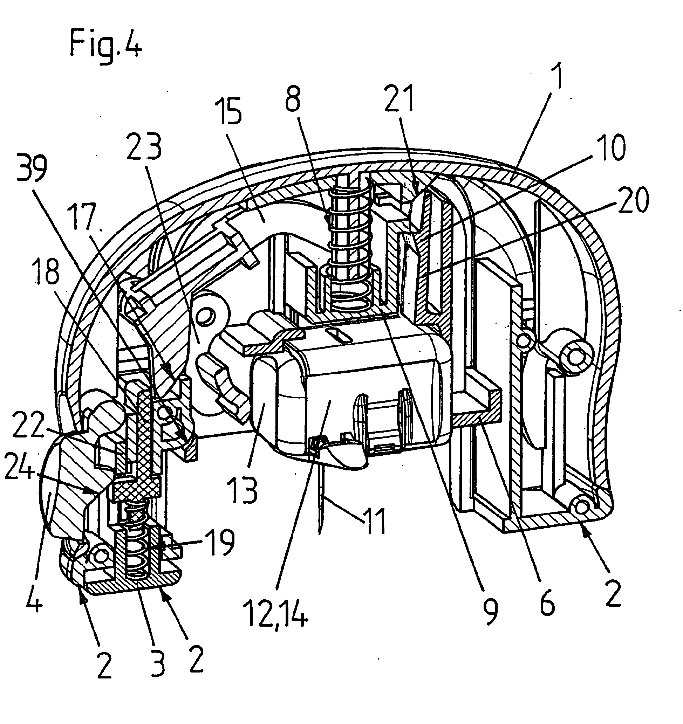

Wie in Zusammenschau mit den Figuren 2, 3 und 4 ersichtlich wird, welche die Insertionsvorrichtung in einem Zustand wie zuvor, jedoch mit in die Aufnahmeöffnung eingesetztem Insertionskopf 12 (Fig. 2), während dem Vorspannen mit dem darin angeordnetem Insertionskopf 12 (Fig. 3) und im vorgespannten, gesicherten und applikationsbereiten Zustand (Fig. 4) zeigen, wird zum Vorspannen und gleichzeitigen Ausklappen der Infusionskanüle 11 des Infusionssets 12 das Gehäuse 1 mit einer Hand und das Schieberelement 6 mit der anderen Hand ergriffen und mit dem darin gehaltenen Infusionsset 12 gegenüber dem Gehäuse 1 nach oben bewegt, unter Mitnahme des Hammerelements 9 und einer daraus resultierenden zunehmenden Vorspannung der Spiralfeder 8. Da das Schieberelement 6 beim Nachobenbewegen lediglich die Portalöffnung der Gehäuses 1 freigibt, jedoch in seiner obersten Position nicht über die Oberkante des Gehäuses 1 übersteht, bleiben die Gesamtabmessungen der Insertionsvorrichtung hierbei unverändert.

Gleichzeitig wird die Kanüle 11 des Insertionskopfes 12 ausgeklappt, indem ein schwenkbar am Schieberelement 6 gelagerter Aktivierungshebel 23 an einer Steuerrampe 39 im Gehäuse 1 anläuft und auf den Insertionskopf 12 zu geschwenkt wird, wobei er den linken Gehäuseteil 13 des Insertionskopfes 12, welcher ein verschiebliches Aktivierungsorgan des Insertionskopfes 12 bildet, in den rechten Gehäuseteil 14 desselben einschiebt und dadurch über einen insertionskopfinternen Mechanismus (nicht gezeigt) die Kanüle 11 ausklappt. Die in diesem und den folgenden Ausführungsbeispielen gezeigten Insertionsköpfe 12 sind ausnahmslos Infusionssets für Insulin, welche in den hier gezeigten Zuständen vor der Applikation eine von einer nach der Applikation zu entfernenden Einstechnadel gestützte flexible Kanüle (Softkanüle) aufweisen. Für die Zwecke der Darlegung der Erfindung muss hier jedoch nicht zwischen Einstechnadel und flexibler Kanüle unterschieden werden, weshalb beide zusammen jeweils als "Kanüle 11" bezeichnet werden.

Ebenfalls gleichzeitig mit der Bewegung des Insertionskopfes 12 mit dem Schieberelement 6 von der in der Fig. 2 dargestellten Aufnahmeposition in die in Fig. 4 dargestellte Bereitschaftsposition, in welcher sich der Insertionskopf 12 in der anspruchsgemässen ersten Position befindet, wird ein als Doppelhebel ausgebildeter Auslöserhebel 15, welcher an seinem ersten Ende mit einem Führungszylinder (nicht gezeigt), einem Gleitstein vergleichbar, in einem horizontalen Langloch 16 an der Rückseite des Hammerelements 9 geführt ist, um eine Lagerstelle im Gehäuse 1 geschwenkt, wobei er mit einer an seinem zweiten Ende ausgebildeten Anlauframpe 17 ein Riegelelement 18 entgegen der Kraft einer Verriegelungsfeder 19 nach unten bewegt, bis er über das Riegelelement 18 hinaus läuft und sodann durch das unter der Federkraft zurückschnellende Riegelelement 18 in der in Fig. 4 dargestellten Situation verriegelt wird. Entsprechend wird in dieser Situation das Hammerelement 9 durch den Auslöserhebel 15 entgegen der Kraft der vorgespannten Feder 8 in der in Fig. 4 gezeigten oberen Position gehalten.

Dabei wird der Rastvorsprung 10, welcher das Hammerelement 9 mitnimmt, sobald der Auslöserhebel 15 durch das Riegelelement 18 verriegelt ist, ausser Eingriff mit dem Hammerelement 9 gebracht, indem die den Rastvorsprung 10 tragende Federlasche 20 des Schieberelements 6 mit ihrem freien Ende an einer Rampenfläche 21 im Gehäuse 1 anläuft, so dass die Lasche 20 vom Hammerelement 6 weg gebogen wird. In diesem Zustand, in welchem das Schieberelement 6 maximal nach oben geschoben ist, verrastet es reversibel mit dem Gehäuse 1 mit (nicht dargestellten) Rastmitteln, derart, dass es nur nach Überwindung eines erhöhten Anfangswiderstandes in die entgegen gesetzte Richtung und wieder in die Einsetzposition bewegt werden kann. Geeignete Rastmittel sind dem Fachmann bekannt und könnten beispielsweise durch eine an eine Federzunge gehaltene Rastnase gebildet werden, welche mit einer Anlaufschräge in einen Hinterschnitt einrastet, so dass die Rastverbindung unter einem mit erhöhtem Kraftaufwand einhergehenden Auslenken der Federzunge durch Anlaufen der Anlaufschräge an einer Kante des Hinterschnitts wieder aufgelöst werden kann.

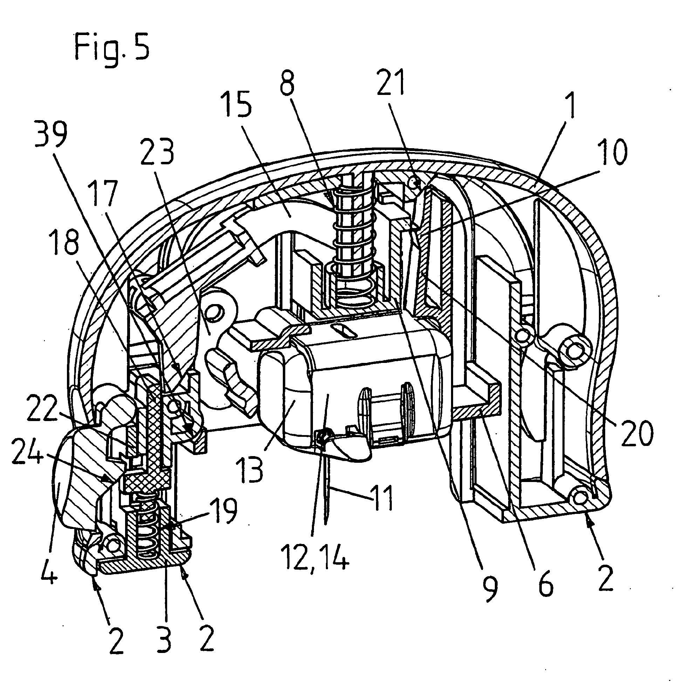

Wie aus einer Zusammenschau der Figuren 4 und 5 hervorgeht, welche die Insertionsvorrichtung jeweils im applikationsbereiten Zustand zeigen, jedoch einmal gesichert (Fig. 4) und einmal entsichert (Fig. 5), bildet der Sicherungstaster 3, auf welchem sich die Verriegelungsfeder 19 mit ihrem dem Riegelelement 18 abgewandten Ende abstützt, im Gehäuse 1 einen Sicherungsschieber 22, welcher in der in Fig. 4 gezeigten Situation eine Betätigbarkeit des Auslöseknopfes 4 formschlüssig verhindert. Erst durch Beaufschlagung des Sicherungstasters 3 mit einer Druckkraft entgegen der Kraftrichtung der Verriegelungsfeder 19, z.B. durch Anpressen der Insertionsvorrichtung an die Applikationsstelle am Körper eines Patienten, kann dieser soweit in das Gehäuse 1 hineingeschoben werden, dass seine eine Kontaktfläche 2 tragenden Unterseite im wesentlichen oberflächenbündig mit der an diese angrenzenden Kontaktfläche 2 des Gehäuses 1 ist. In dieser Position, welche in Fig. 5 dargestellt ist, gibt der Sicherungsschieber 22 den Auslöseknopf 4 frei.

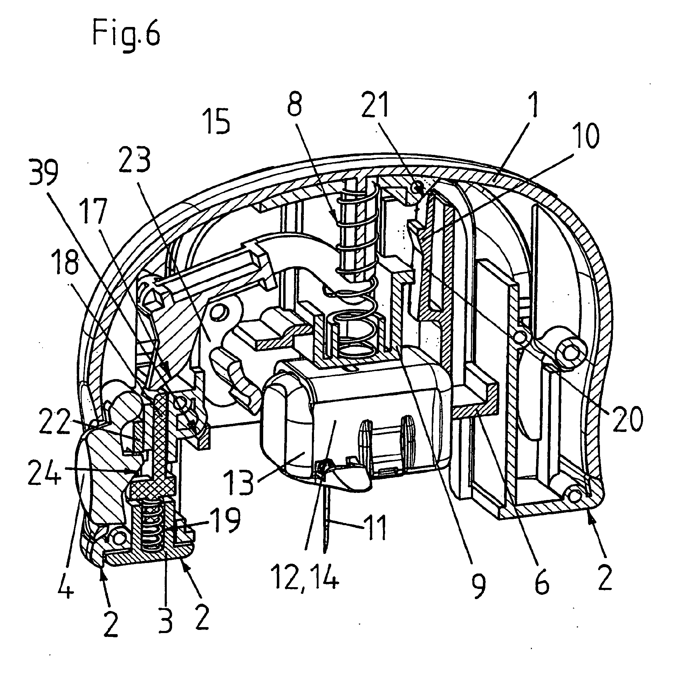

Wie aus Fig. 6 hervorgeht, welche die Insertionsvorrichtung kurz nach dem Betätigen des Auslöseknopfes 4 zeigt, weist der Auslöseknopf 4 auf seiner dem Inneren des Gehäuses 1 zugewandten Seite eine Auslöserampe 24 auf, welche beim Betätigen des Auslöseknopfes 4 entlang einer Steuerkante des Riegelelements 18 bewegt wird und dieses dadurch entgegen der Kraft der Verriegelungsfeder 19 nach unten zieht, wodurch das verriegelte zweite Ende des Auslöserriegels 15 freigegeben wird und das an dessen erstem Ende gehaltene Hammerelement 9 durch die Kraft der vorgespannten Spiralfeder 8 getrieben nach unten schnellt. Dabei trifft das Hammerelement 9 auf die Oberseite des kraftschlüssig in der anspruchsgemässen ersten Position im Schieberelement 6 gehaltenen Insertionskopfes 12, löst diesen von den Rastvorsprüngen 7 und treibt diesen, beim Applizieren des Insertionskopfes 12 am Körper eines Patienten, unter einem Einstechen der Kanüle 11 in den Körper weiter vor sich her, bis die Kanüle 11 vollständig eingestochen und der Insertionskopf 12 mit seiner Unterseite auf der Körperoberfläche aufliegt. Dabei verbleibt das Schieberelement 6 mit den Rastvorsprüngen 7 unbewegt gegenüber dem Gehäuse 1 und muss für die. Applikation eines weiteren Insertionskopfes 12 zuerst wieder in die Einsetzposition zurückgebracht werden, indem es unter Überwindung eines anfänglich erhöhten Widerstandes gegenüber dem Gehäuse 1 nach unten bewegt wird.

Nach dem Applizieren ist der applizierte Insertionskopf 12 vollständig von der Insertionsvorrichtung getrennt, so dass diese ohne jegliche Irritation der Applikationsstelle entfernt werden kann.

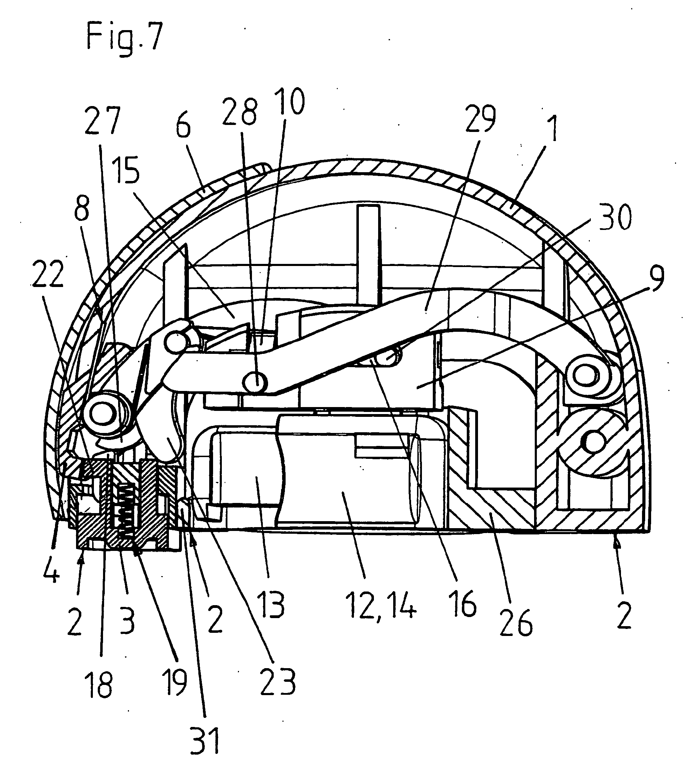

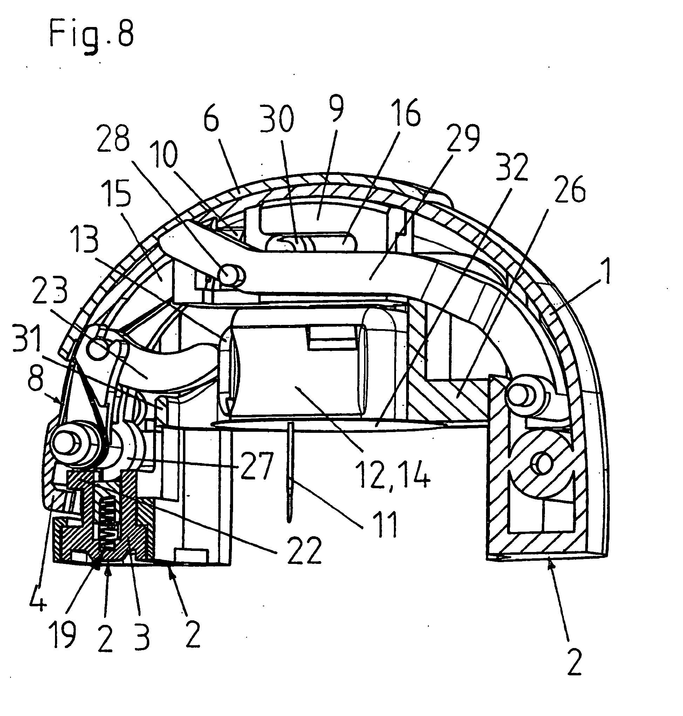

Eine zweite Ausführungsform der erfindungsgemässen Insertionsvorrichtung, ebenfalls für ein Infusionsset mit ausklappbarer Infusionskanüle, ist in den Figuren 7 und 8 jeweils im Vertikalschnitt dargestellt, und zwar einmal im nicht vorgespannten Zustand mit bereits darin eingesetztem Insertionskopf (Fig. 7) und einmal im vorgespannten, entsicherten Zustand mit darin applikationsbereit in der anspruchsgemässen ersten Position angeordnetem Infusionsset (Fig. 8).

Wie zu erkennen ist, weist auch diese Insertionsvorrichtung ein portalartiges Gehäuse 1 auf, welches an seiner Unterseite Kontaktflächen 2 zum Aufsetzen und Andrücken der Insertionsvorrichtung auf den Körper eines Patienten beim Applizieren eines Infusionssets mit der Insertionsvorrichtung aufweist. Ein Teil der Kontaktflächen 2 wird von einem Sicherungstaster 3 gebildet, welcher an der Unterseite des Gehäuses 1 nach unten vorsteht und zum Entsichern der Insertionsvorrichtung, wenn sich diese in einem applikationsbereiten Zustand befindet, durch Aufsetzen auf und Andrücken an den Körper des Patienten in eine Entsicherungsposition verschoben werden kann, in welcher die Kontaktfläche 2 des Sicherungstasters 3 im Wesentlichen oberflächenbündig mit der an den Sicherungstaster 3 angrenzenden Kontaktfläche 2 des Gehäuses 1 ist (siehe Fig.8).

Weiter weist die Insertionsvorrichtung einen Auslöseknopf 4 auf, mit welchem bei in dem Zustand gemäss Fig. 8 sich befindlicher Insertionsvorrichtung die Insertionsbewegung zum Applizieren des Insertionskopfes am Körper des Patienten ausgelöst werden kann. Sicherungstaster 3 und Auslöseknopf 4 stellen auch hier zwei anspruchsgemässe Betätigungsorgane 3, 4 dar, welche zur Auslösung der Insertionsbewegung gleichzeitig betätigt sein müssen.

Auch diese Insertionsvorrichtung weist im entspannten, insertionskopffreien Zustand (wie Fig. 7, jedoch ohne eingesetztes Infusionsset) auf ihrer Unterseite eine Aufnahmeöffnung auf, welche von einem im Gehäuse 1 vertikal verschiebbar gelagerten Aufnahmeelement 26 gebildet wird und in welcher vom Aufnahmeelement 26 federelastisch gelagerte Vorsprünge (nicht gezeigt) bereitgestellt werden, mit denen das Infusionsset 12 in beiden dargestellten Situationen ausschliesslich kraftschlüssig in der Aufnahmeöffnung gehalten wird.

Wie weiter zu erkennen ist, umfasst die Insertionsvorrichtung als Antriebsmittel eine Drehfeder 8, die über einen Auslöserhebel 15 auf ein Hammerelement 9 wirkt. Der Auslöserhebel 15 ist drehbar um das Drehzentrum der Drehfeder 8 im Gehäuse gelagert und weist an seinem drehzentrumfernen Ende einen Führungszylinder 30 auf, welcher einem Gleitstein ähnlich in einem horizontalen Langloch 16 im Hammerelement 9 geführt ist.

Zum Vorspannen und gleichzeitigem Ausklappen der Infusionskanüle 11 des im Aufnahmeelement 26 gehaltenen Infusionssets 12 wird das Gehäuse 1 mit einer Hand und ein entlang dessen Aussenkontur verschiebbares Schieberelement 6 mit der anderen Hand ergriffen und sodann das Schieberelement 6 von der in Fig. 7 gezeigten Position in die in Fig. 8 gezeigte Position verschoben. Ebenso ist es möglich, das Schieberelement 6 mit der gleichen Hand zu bedienen, mit welcher das Gehäuse 1 gehalten wird.

Wie aus den Figuren aufgrund der Schnittlegung nicht ersichtlich ist, wird bei der Verschiebung des Schieberelements 6 eine von diesem gebildete Kulissenführung (nicht gezeigt) entlang eines Führungszylinders 28 eines Spannhebels 29 geführt, wodurch der Spannhebel 29 nach oben geführt wird. Auf der Hinterseite weist der Spannhebel 29 gegenüberliegend dem Führungszylinder 28 einen weiteren Zylinder (nicht sichtbar) auf, welcher unter eine im Hammerelement 9 gelagerte Sperrklinke 10 greift.. Bei seiner Nachobenbewegung nimmt der Spannhebel 29 über den Zylinder und die Sperrklinke 10 das Hammerelement 9 mit nach oben, welches wiederum über das Langloch 16 und den Führungszylinder 30 den Auslöserhebel 15 entgegen der Kraft der vorgespannten Drehfeder 8 unter zunehmender Vorspannung derselben von der in der Fig. 7 dargestellten Position in die in Fig. 8 dargestellte Position nach oben mitnimmt. Dabei gleitet der Zylinder des Spannhebels 29 horizontal entlang der Unterseite der Sperrklinke 10, bis er am Ende der Vorspannbewegung neben der Sperrklinke 10 angeordnet ist, also ausser Eingriff mit der Klinke 10 steht.

Wie in Fig. 7 erkennbar ist, weist der Auslöserhebel 15 an seinem das Drehzentrum bildenden Ende ein Verriegelungselement 27 auf, welches bei seiner Schwenkbewegung gleitend an einem mit der Kraft einer Verriegelungsfeder 19 beaufschlagtem Riegelelement 18 entlang geführt wird, bis es über das Riegelelement 18 hinaus läuft und sodann durch dieses in der in Fig. 8 dargestellten vorgespannten Situation verriegelt wird. Dieses Verriegeln erfolgt kurz bevor der Zylinder des Spannhebels 29 ausser Eingriff mit der Sperrklinke 10 gerät. Nach dem Aussereingriffbringen des Zylinders von der Sperrklinke 10 wird das Hammerelement 9 durch den Auslöserhebel 15 in der in Fig. 8 gezeigten oberen Position gehalten.

Weiter nimmt das Hammerelement 9 bei seiner Nachobenbewegung das Schieberelement 26 mit, welches wiederum mit einer von diesem gebildeten Mitnehmernase 31 einen drehbar entgegen der Kraft einer Hilfsfeder am Auslöserhebel 15 gelagerten Aktivierungshebel 23 mitnimmt. Dabei wird der Aktivierungshebel 23 auf den Insertionskopf 12 zu geschwenkt, wobei er den linken Gehäuseteil 13 des Insertionskopfes 12, welcher ein verschiebliches Aktivierungsorgan des Insertionskopfes 12 bildet, in den rechten Gehäuseteil 14 desselben einschiebt und dadurch über einen insertionskopfinternen Mechanismus (nicht gezeigt) die Kanüle 11 ausklappt.

Kurz bevor der Zylinder des Spannhebels 29 ausser Eingriff mit der Sperrklinke 10 kommt, verrastet das Schieberelement 26 in der in Fig. 8 gezeigten Position reversibel mit dem Gehäuse 1 mit (nicht dargestellten) Rastmitteln, derart, dass es nur nach Überwindung eines erhöhten Anfangswiderstandes in die entgegengesetzte Richtung und wieder in die Einsetzposition bewegt werden kann. Geeignete Rastmittel sind dem Fachmann bekannt und könnten auch hier beispielsweise durch eine an eine Federzunge gehaltene Rastnase gebildet werden, welche mit einer Anlaufschräge in einen Hinterschnitt einrastet, so dass die Rastverbindung unter einem mit erhöhtem Kraftaufwand einhergehenden Auslenken der Federzunge durch Anlaufen der Anlaufschräge an einer Kante des Hinterschnitts wieder aufgelöst werden kann.

Wie aus einer Zusammenschau der Figuren 7 und 8 hervorgeht, bildet der Sicherungstaster 3, auf welchem sich die Verriegelungsfeder 19 mit ihrem dem Riegelelement 18 abgewandten Ende abstützt, auch hier im Gehäuse 1 einen Sicherungsschieber 22, welcher in der in Fig. 7 gezeigten Situation eine Betätigbarkeit des Auslöseknopfes 4 formschlüssig verhindert. Erst durch Beaufschlagung des Sicherungstasters 3 mit einer Druckkraft entgegen der Kraftrichtung der Verriegelungsfeder 19, z.B. durch Anpressen der Insertionsvorrichtung an die Applikationsstelle am Körper eines Patienten, kann dieser soweit in das Gehäuse 1 hineingeschoben werden, dass seine eine Kontaktfläche 2 tragenden Unterseite im Wesentlichen oberflächenbündig mit der an diese angrenzenden Kontaktfläche 2 des Gehäuses 1 ist. In dieser Position, welche in Fig. 8 dargestellt ist, gibt der Sicherungsschieber 22 den Auslöseknopf 4 frei.

Auch bei dieser Ausführungsform weist der Auslöseknopf 4 auf seiner dem Inneren des Gehäuses 1 zugewandten Seite eine Auslöserampe (nicht sichtbar) auf, welche beim Betätigen des Auslöseknopfes 4 entlang einer Steuerkante des Riegelelements 18 bewegt wird und dieses dadurch entgegen der Kraft der Verriegelungsfeder 19 nach unten zieht, wodurch das Verriegelungselement 27 des Auslöserhebels 15 freigegeben wird und das an dessen freiem Ende gehaltene Hammerelement 9 durch die Kraft der vorgespannten Drehfeder 8 getrieben nach unten schnellt. Dabei trifft das Hammerelement 9 auf die Oberseite des kraftschlüssig in der anspruchsgemässen ersten Position im Schieberelement 6 gehaltenen Insertionskopfes 12, löst diesen von den Rastvorsprüngen und treibt diesen, beim Applizieren des Insertionskopfes 12 am Körper eines Patienten, unter einem Einstechen der Kanüle 11 in den Körper weiter vor sich her, bis die Kanüle 11 vollständig eingestochen ist und der Insertionskopf 12 mit seiner Unterseite, welche von einem Klebepad 32 gebildet ist, auf der Körperoberfläche aufliegt. Dabei verbleibt das Aufnahmeelement 26 unbewegt gegenüber dem Gehäuse 1 und muss für die Applikation eines weiteren Insertionskopfes 12 zuerst wieder in die Einsetzposition zurückgebracht werden. Hierzu wird der Schieber 6 zurück in die in Fig. 7 gezeigte Position gebracht, wobei er den Spannhebel 29 wieder nach unten mitnimmt, welcher wiederum das Aufnahmeelement 26 unter Überwindung eines anfänglich erhöhten Widerstandes aus der Verrastung mit dem Gehäuse 1 löst und dieses sodann in die in Fig. 7 gezeigte Position mitnimmt. Bei der Nachuntenbewegung des Spannhebels 29 überläuft der auf seiner Rückseite angeordnete Zylinder (nicht sichtbar) die Sperrklinke 10, drückt diese vorübergehend gegen die Kraft einer Sperrklinkenfeder (nicht gezeigt) nach hinten und verrastet anschliessend wieder unter dieser.. In diesem Zustand kann ein weiteres Infusionsset in die Aufnahme des Aufnahmeelements 26 eingesetzt werden.

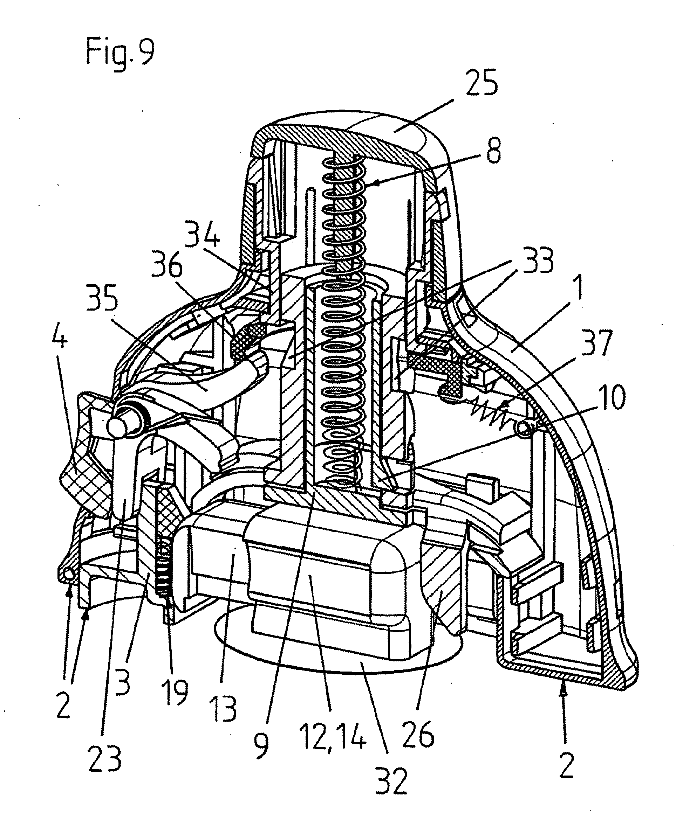

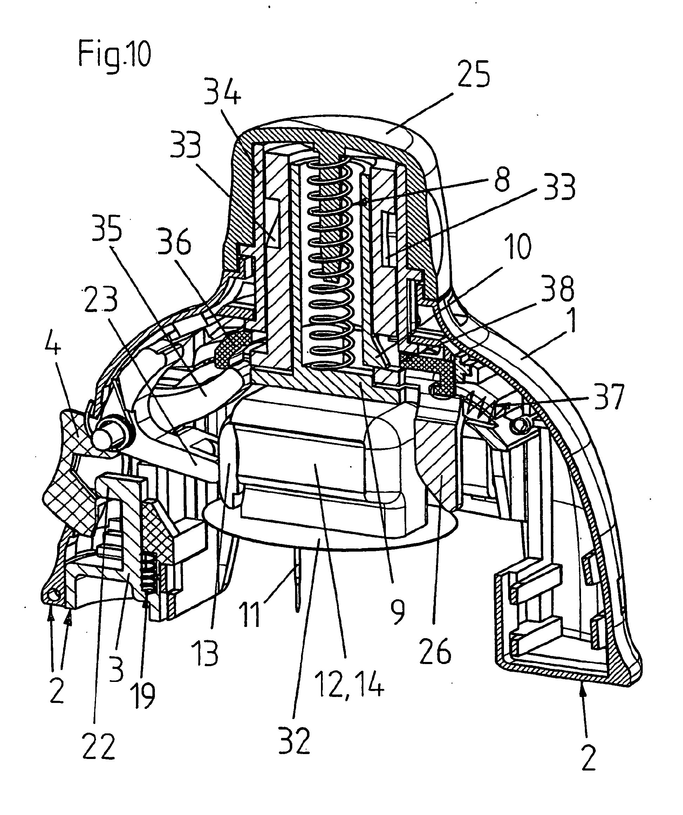

Eine dritte Ausführungsform der erfindungsgemässen Insertionsvorrichtung., ebenfalls für ein Infusionsset mit ausklappbarer Infusionskanüle, ist in den Figuren 9 und 10 jeweils im Vertikalschnitt dargestellt, und zwar einmal im nicht vorgespannten Zustand mit bereits darin eingesetztem Insertionskopf (Fig. 9) und einmal im vorgespannten, entsicherten Zustand mit darin applikationsbereit in der anspruchsgemässen ersten Position angeordnetem Infusionsset (Fig. 10).

Wie zu erkennen ist, weist auch diese dritte Insertionsvorrichtung ein portalartiges Gehäuse 1 auf, welches an seiner Unterseite Kontaktflächen 2 zum Aufsetzen und Andrücken der Insertionsvorrichtung auf den Körper eines Patienten beim Applizieren eines Infusionssets mit der Insertionsvorrichtung aufweist. Eine der Kontaktflächen 2 wird wiederum von einem Sicherungstaster 3 gebildet, welcher, wie schon bei den beiden zuvor beschriebenen Insertionsvorrichtungen an der Unterseite des Gehäuses 1 nach unten vorsteht und zum Entsichern der Insertionsvorrichtung, wenn sich diese in einem applikationsbereiten Zustand befindet, durch Aufsetzen auf und Andrücken an den Körper des Patienten in eine Entsicherungsposition verschoben werden kann, in welcher die Kontaktfläche 2 des Sicherungstasters 3 im Wesentlichen oberflächenbündig mit der an den Sicherungstaster 3 angrenzenden Kontaktfläche 2 des Gehäuses 1 ist (siehe Fig. 10).

Auch hier weist die Insertionsvorrichtung einen Auslöseknopf 4 auf, mit welchem bei in dem Zustand gemäss Fig. 10 sich befindlicher Insertionsvorrichtung die Insertionsbewegung zum Applizieren des Insertionskopfes am Körper des Patienten ausgelöst werden kann. Sicherungstaster 3 und Auslöseknopf 4 stellen auch hier zwei anspruchsgemässe Betätigungsorgane 3, 4 dar, welche zur Auslösung der Insertionsbewegung gleichzeitig betätigt sein müssen.

Auch diese Insertionsvorrichtung weist im nicht vorgespannten, insertionskopffreien Zustand (wie Fig. 9, jedoch ohne eingesetztes Infusionsset) auf ihrer Unterseite eine Aufnahmeöffnung auf, welche von einem im Gehäuse 1 vertikal verschiebbar gelagerten Aufnahmeelement 26 gebildet wird und in welcher vom Aufnahmeelement 26 gebildete Haltemittel (nicht gezeigt) angeordnet sind, mit denen das Infusionsset 12 kraftschlüssig in der Aufnahmeöffnung gehalten wird.

Wie weiter zu erkennen ist, umfasst die Insertionsvorrichtung wie schon die erste beschriebene Ausführungsform als Antriebsmittel eine Spiralfeder 8, die direkt auf ein Hammerelement 9 wirkt, welches sich in den dargestellten Situationen mit einem Rastvorsprung 10 auf dem Aufnahmeelement 26 abstützt. Die Spiralfeder 8 wird koaxial von im Wesentlichen zylindrisch ausgebildeten Abschnitten des Hammerelements 9 und des Aufnahmeelements 26 umgeben. Dabei weist der zylinderförmige Abschnitt des Aufnahmeelements 26 an seinem Aussenumfang spiralabschnittsförmige Führungskulissen 33 auf, in welche Gleitsteine (nicht sichtbar) einer diesen Abschnitt des Aufnahmeelements 26 umgebenden Drehhülse 34 eingreifen. Die Drehhülse 34 wiederum ist verdrehbar aber axial bezogen auf die Kraftrichtung der Feder 8 unverschiebbar im Gehäuse 1 gelagert und trägt einen Drehknopf 25, an dessen Innenseite sich die Spiralfeder 8 abstützt.

Zum Vorspannen und gleichzeitigem Ausklappen der Infusionskanüle 11 des im Aufnahmeelement 26 gehaltenen Infusionssets 12 wird das Gehäuse 1 der sich im Zustand gemäss Fig. 9 befindlichen Insertionsvorrichtung mit einer Hand ergriffen und mit der anderen Hand der Drehknopf 25 um etwa 120° verdreht, was zu einer entsprechenden Verdrehung der Drehhülse 34 gegenüber dem Gehäuse 1 führt. Hierdurch werden deren Gleitsteine innerhalb der Führungskulissen 33 des Aufnahmeelements 26 verschoben und als Resultat das Aufnahmeelement 26 mit dem darin gehaltenen Infusionsset 12 von der in Fig. 9 gezeigten Position in die in Fig. 10 gezeigte Position angehoben, unter Mitnahme des Hammerelements 9 und entsprechender zunehmender Vorspannung der Spiralfeder 8. Bei Erreichen seiner Drehposition, welche der in Fig. 10 dargestellten maximal vorgespannten Situation des Insertionsvorrichtung entspricht, verrastet der Drehknopf 25 in dieser Drehposition reversibel mit dem Gehäuse 1 mit (nicht dargestellten) Rastmitteln, derart, dass er nur nach Überwindung eines erhöhten Anfangswiderstandes weitergedreht oder in die entgegen gesetzte Richtung gedreht werden kann. Geeignete Rastmittel sind dem Fachmann bekannt und könnten beispielsweise durch eine an eine Federzunge gehaltene Rastnase gebildet werden, welche mit einer Anlaufschräge in einen Hinterschnitt einrastet, so dass die Rastverbindung unter einem mit erhöhtem Kraftaufwand einhergehenden Auslenken der Federzunge durch Anlaufen der Anlaufschräge an einer Kante des Hinterschnitts wieder aufgelöst werden kann.

Da das Halteelement 26 beim Nachobenbewegen lediglich die Portalöffnung der Gehäuses 1 freigibt, jedoch innerhalb des Gehäuses 1 verbleibt, bleiben auch hier, wie schon bei der ersten Ausführungsform der erfindungsgemässen Insertionsvorrichtung, die Gesamtabmessungen der Insertionsvorrichtung unverändert.

Gleichzeitig mit dem Verdrehen des Drehknopfes 25 und der dadurch bewirkten Bewegung der internen Komponenten 8, 9, 26, 34 wird die Kanüle 11 des Insertionskopfes 12 ausgeklappt, indem einer der beiden Arme eines im Gehäuse 1 gelagerten, als Doppelhebel ausgebildeten Aktivierungshebels 23 von dem Aufnahmeelement 26 mitgenommen wird (aufgrund der Schnittlegung nicht erkennbar) und dadurch dessen zweiter Arm auf den Insertionskopf 12 zu geschwenkt wird, wobei er den linken Gehäuseteil 13 des Insertionskopfes 12, welcher ein verschiebliches Aktivierungsorgan des Insertionskopfes 12 bildet, in den rechten Gehäuseteil 14 desselben einschiebt und dadurch über einen insertionskopfinternen Mechanismus (nicht gezeigt) die Kanüle 11 ausklappt.

Wie aus einer Zusammenschau der Figuren 9 und 10 hervorgeht, wird der Sicherungstaster 3 durch die Kraft einer Feder 19 nach aussen gedrückt und bildet im Gehäuse 1 einen Sicherungsschieber 22, welcher in der in Fig. 9 gezeigten Situation eine Betätigbarkeit des Auslöseknopfes 4 formschlüssig verhindert. Erst durch Beaufschlagung des Sicherungstasters 3 mit einer Druckkraft entgegen der Kraftrichtung der Feder 19, z.B. durch Anpressen der Insertionsvorrichtung an die Applikationsstelle am Körper eines Patienten, kann dieser soweit in das Gehäuse 1 hineingeschoben werden, dass seine eine Kontaktfläche 2 tragenden Unterseite im wesentlichen oberflächenbündig mit der an diese angrenzenden Kontaktfläche 2 des Gehäuses 1 ist. In dieser Position, welche in Fig. 10 dargestellt ist, gibt der Sicherungsschieber 22 den Auslöseknopf 4 frei.

Der Auslöseknopf 4 ist schwenkbar um die Drehachse des Aktivierungshebels 23 im Gehäuse 1 gelagert und bildet innerhalb des Gehäuses 1 einen Auslöserhebel 35, welcher an einer Steuerkante einer im Gehäuse 1 horizontal verschiebbar und axial unverschiebbar bezüglich der Kraftrichtung der Antriebsfeder 8 gelagerten Auslösekulisse 36 anläuft, derart, dass eine Betätigung des Auslöseknopfes 4 die Auslösekulisse 36 entgegen der Kraft eine Rückstellfeder 37 nach links verschiebt.

Hierdurch wird, bei vorgespannter Insertionsvorrichtung (Fig.. 10) der Rastvorsprung 10, mit welchem sich das mit der Kraft der Spiralfeder 8 beaufschlagte Hammerelement 9 auf dem Aufnahmeelement 26 abstützt, nach links gedrückt und gerät dadurch ausser Eingriff mit dem Aufnahmeelement 26, so dass das Hammerelement 9 durch die Kraft der Feder 8 getrieben und innerhalb des zylindrischen Abschnitts des Halteelements 26 geführt nach unten schnellt. Dabei trifft das Hammerelement 9 auf die Oberseite des kraftschlüssig in der anspruchsgemässen ersten Position im Halteelement 26 gehaltenen Insertionskopfes 12, löst diesen von den Haltemitteln und treibt diesen, beim Applizieren des Insertionskopfes 12 am Körper eines Patienten, unter einem Einstechen der Kanüle 11 in den Körper weiter vor sich her, bis die Kanüle 11 vollständig eingestochen ist und der Insertionskopf 12 mit seiner Unterseite, welche hier von einem Klebepflaster 32 gebildet wird, auf der Körperoberfläche aufliegt. Dabei verbleibt das Halteelement 26 mit den Haltemitteln unbewegt gegenüber dem Gehäuse 1 in der oberen Position und muss für die Applikation eines weiteren Insertionskopfes 12 zuerst wieder in die Einsetzposition zurückgebracht werden, indem der Drehknopf 25 unter Überwindung eines anfänglich erhöhten Widerstandes gegenüber dem Gehäuse 1 verdreht wird. Da der zylinderförmige Abschnitt des Halteelements 26 spiralabschnittsförmige Kulissenführungen 33 aufweist, welche abwechselnd links und rechtsdrehend sind, ist es egal, ob der Drehknopf 25 zur Überführung der Insertionsvorrichtung von der Einsetzposition in die Bereitschaftsposition oder zum Zurückstellen des Halteelements nach der Applikation rechts- oder linksherum gedreht wird, da eine Verdrehung, egal in welche Richtung, jeweils zum angestrebten Zustand führt.

Auch wenn vorstehend drei verschiedene konkrete Ausführungsformen der erfindungsgemässen Insertionsvorrichtung beschrieben wurden, sei darauf hingewiesen, dass die in den verschiedenen Ausführungsbeispielen gezeigten technischen Lösungen natürlich auch miteinander kombiniert werden können, um weitere erfindungsgemässe Ausführungsformen der Insertionsvorrichtung zu bilden.

## Patentansprüche

1. Insertionsvorrichtung für einen Insertionskopf (12) mit mindestens einer Infusionskanüle (11) und/oder mit mindestens einer Einstechspitze zum Einstechen in den Körper eines Patienten, umfassend
a) mindestens eine Kontaktfläche (2) zum Aufsetzen der Insertionsvorrichtung auf eine Applikationsstelle am Körper des Patienten beim Applizieren des Insertionskopfes (12),
b) Haltemittel (6, 7) zum vorübergehenden Halten des zu applizierenden Insertionskopfes (12) an der Insertionsvorrichtung,
c) Antriebsmittel (8, 9) zum Bewirken einer Insertionsbewegung des Insertionskopfes (12) relativ zu der Kontaktfläche (2) in Längsrichtung einer Infusionskanüle (11) oder Einstechspitze des applikationsbereiten Insertionskopfes (12) von einer ersten Position, in welcher der applikationsbereite Insertionskopf (12) mit den Haltemitteln (6, 7) derart gehalten ist, dass sämtliche Infusionskanülen (11) und Einstechspitzen des Insertionskopfes (12) gegenüber der Kontaktfläche (2) zurückstehen, in eine zweite Position, in welcher sämtliche Infusionskanülen (11) und Einstechspitzen des Insertionskopfes (12) im wesentlichen vollständig über die Kontaktfläche (2) hinaus stehen, zur Ermöglichung eines Einstechens sämtlicher Infusionskanülen (11) und Einstechspitzen des Insertionskopfes (12) in den Körper des Patienten beim Ausführen der Insertionsbewegung bei mit der Kontaktfläche (2) auf den Körper aufgesetzter Insertionsvorrichtung,
wobei die Antriebsmittel (8, 9) mindestens ein vorspannbares Kraftspeicherelement (8) zur Bereitstellung der Antriebsenergie aufweisen und die Haltemittel (6, 7) relativ zur Kontaktfläche (2) in einer Einsetzposition und in einer Bereitschaftsposition positionierbar sind und derartig ausgestaltet sind, dass der zu applizierende Insertionskopf (12) in der Einsetzposition an den Haltemitteln (6, 7) angeordnet werden kann, derart, dass er von diesen gehalten wird, und die Haltemittel (6, 7) sodann ausgehend von der Einsetzposition mit dem daran gehaltenen Insertionskopf (12) in die Bereitschaftsposition bringbar sind, wobei der Insertionskopf (12) bei Erreichen der Bereitschaftsposition von den Haltemitteln (6, 7) applikationsbereit in der ersten Position gehalten wird, wobei die Insertionsvorrichtung derartig ausgebildet ist, dass der Insertionskopf (12) zu Beginn der Insertionsbewegung von den Haltemitteln (6, 7) separiert wird, derart, dass dieser zumindest den grössten Teil der Insertionsbewegung losgelöst von den Haltemitteln (6, 7) vollführen kann, und wobei die Insertionsvorrichtung weiter derartig ausgebildet ist, dass das vorspannbare Kraftspeicherelement (8) beim Bewegen der Haltemittel (6, 7) von der Einsetzposition in die Bereitschaftsposition vorgespannt wird.

2. Insertionsvorrichtung nach Anspruch 1, **dadurch gekennzeichnet, dass** die Insertionsvorrichtung derartig ausgebildet ist, dass der Insertionskopf (12) bei in der Bereitschaftsposition angeordneten Haltemitteln (6, 7) in der ersten Position rein kraftschlüssig, rein formschlüssig oder kraft- und formschlüssig von den Haltemitteln (6, 7) gehalten werden kann.

3. Insertionsvorrichtung nach Anspruch 2, **dadurch gekennzeichnet, dass** die Insertionsvorrichtung derartig ausgebildet ist, dass der Insertionskopf (12) bei in der Einsetzposition angeordneten Haltemitteln (6, 7) rein formschlüssig oder kraft- und formschlüssig von den Haltemitteln (6, 7) gehalten werden kann, und insbesondere, wobei der Formschluss beim Bewegen der Haltemittel (6, 7) mit dem darin gehaltenen Insertionskopf (12) von der Einsetzposition in die Bereitschaftsposition aufgehoben wird.

4. Insertionsvorrichtung nach Anspruch 2, **dadurch gekennzeichnet, dass** die Insertionsvorrichtung derartig ausgebildet ist, dass der Insertionskopf (12) bei in der Einsetzposition angeordneten Haltemitteln rein kraftschlüssig von den Haltemitteln (6, 7) gehalten werden kann, und insbesondere, wobei der Kraftschluss beim Bewegen der Haltemittel (6, 7) mit dem darin gehaltenen Insertionskopf (12) von der Einsetzposition in die Bereitschaftsposition verringert wird.

5. Insertionsvorrichtung nach einem der vorangehenden Ansprüche, **dadurch gekennzeichnet, dass** die Insertionsvorrichtung derartig ausgebildet ist, dass die Haltemittel (6, 7) zumindest während eines Grossteils der Insertionsbewegung oder während der gesamten Insertionsbewegung des Insertionskopfes (12) unbewegt gegenüber der Kontaktfläche (2) sind.

6. Insertionsvorrichtung nach einem der vorangehenden Ansprüche, **dadurch gekennzeichnet, dass** die Insertionsvorrichtung manuell betätigbare Aktivierungsmittel (6) aufweist, insbesondere ein Schieberelement (6), einen Drehknopf (25) oder einen Drucktaster, mittels welcher die Haltemittel (6, 7) mit dem daran gehaltenen Insertionskopf (12) manuell durch Muskelkraft von der Einsetzposition in die Bereitschaftsposition gebracht werden können, unter einem Vorspannen des Kraftspeicherelements (8).

7. Insertionsvorrichtung nach Anspruch 6, **dadurch gekennzeichnet, dass** die Insertionsvorrichtung ein Gehäuse (1) umfasst und die Haltemittel (6, 7) insbesondere fest mit einem Schieberelement (6) verbunden sind, welches von Hand ergreifbar und gegenüber dem Gehäuse (1) bewegbar, insbesondere verschiebbar ist, zum Bewegen der Haltemittel (6, 7) von der Einsetzposition in die Bereitschaftsposition unter einem Vorspannen des Kraftspeicherelements (8).

8. Insertionsvorrichtung nach einem der vorangehenden Ansprüche, **dadurch gekennzeichnet, dass** die Insertionsvorrichtung derartig ausgebildet ist, dass ihre Gesamtabmessungen beim Bewegen der Haltemittel (6, 7) von der Einsetzposition in die Bereitschaftsposition unverändert bleiben.

9. Insertionsvorrichtung nach einem der vorangehenden Ansprüche, **dadurch gekennzeichnet, dass** die Insertionsvorrichtung derartig ausgestaltet ist, dass das Kraftspeicherelement (8) wiederholt vorspannbar ist, zur Ermöglichung einer wiederholten Benutzung der Insertionsvorrichtung zum Applizieren eines Insertionskopfes (12).

10. Insertionsvorrichtung nach einem der vorangehenden Ansprüche, **dadurch gekennzeichnet, dass** die Insertionsvorrichtung derartig ausgestaltet ist, dass das Kraftspeicherelement (8) bei in der ersten Position befindlichem Insertionskopf (12) im vorgespannten Zustand bereitstellbar ist und sodann die Insertionsbewegung durch Betätigen mindestens eines Betätigungsorgans (3, 4) auslösbar ist, unter zunehmender Entspannung des Kraftspeicherelements (8).

11. Insertionsvorrichtung nach Anspruch 10, **dadurch gekennzeichnet, dass** die Antriebsmittel (8, 9) ein Vortriebselement (9) zur Übertragung der Antriebskraft auf den zu applizierenden Insertionskopf (12) umfassen und derartig ausgestaltet sind, dass das Kraftspeicherelement (8) unter einem Verschieben des Vortriebselements (9) entgegen der Richtung der Insertionsbewegung und unter anschliessendem Verrasten desselben mit durch die Betätigungsorgane (3, 4) lösbaren Rastmitteln (15, 18) vorspannbar und in vorgespanntem Zustand bereitstellbar ist.

12. Insertionsvorrichtung nach einem der Ansprüche 10 bis 11, **dadurch gekennzeichnet, dass** die Insertionsvorrichtung mindestens zwei Betätigungsorgane (3, 4) aufweist, welche zur Auslösung der Insertionsbewegung gleichzeitig betätigt sein müssen, wobei insbesondere ein erstes der Betätigungsorgane (3) derartig ausgebildet ist, dass es durch ein Andrücken der Insertionsvorrichtung mit der Kontaktfläche (2) an den Körper des Patienten, insbesondere in Richtung der Insertionsbewegung des Insertionskopfes (12), betätigbar ist.

13. Insertionsvorrichtung nach Anspruch 12, **dadurch gekennzeichnet, dass** das erste Betätigungsorgan (3) als schieber- oder tasterförmiges Element (3) ausgebildet ist, welches insbesondere sämtliche Kontaktflächen (2) bildet.

14. Insertionsvorrichtung nach einem der Ansprüche 12 bis 13, **dadurch gekennzeichnet, dass** ein zweites Betätigungsorgan (4) als tasten- oder knopfförmiges Element (4) ausgebildet ist, welches durch Druckbeaufschlagung mit einer Fingerkuppe des Bedieners betätigbar ist, insbesondere in einer Richtung quer, insbesondere senkrecht zur bestimmungsgemässen Andrückrichtung der Insertionsvorrichtung an der Körper des Patienten.

15. Insertionsvorrichtung nach einem der Ansprüche 12 bis 14, **dadurch gekennzeichnet, dass** die mindestens zwei Betätigungsorgane (3, 4) derartig miteinander wirkverbunden sind, dass durch die Betätigung des einen Betätigungsorgans (3) eine Sperrung des anderen Betätigungsorgans (4) aufhebbar ist.

16. Insertionsvorrichtung nach einem der Ansprüche 10 bis 15, **dadurch gekennzeichnet, dass** das Betätigungsorgan oder die Betätigungsorgane (3, 4) derartig ausgebildet sind, dass diese mit einer Hand betätigbar sind, zur Ermöglichung eines einhändigen Auslösens der Insertionsbewegung.

17. Insertionsvorrichtung nach einem der Ansprüche 10 bis 16, **dadurch gekennzeichnet, dass** die Betätigungsorgane (3, 4) derartig ausgebildet sind, dass sie bei Wegfall einer Betätigungskraft automatisch wieder in einen unbetätigten Zustand übergehen.

18. Insertionsvorrichtung nach einem der vorangehenden Ansprüche, **dadurch gekennzeichnet, dass** das Kraftspeicherelement (8) eine Spiral-, Schenkel- oder Blattfeder aus Metall oder Kunststoff ist, ein Gummifederelement oder eine Gasdruckfeder.

19. Insertionsvorrichtung nach einem der vorangehenden Ansprüche, **dadurch gekennzeichnet, dass** die Insertionsvorrichtung Mittel (23) zur Bewirkung einer Verschiebung, insbesondere einer Querverschiebung, eines verschieblichen Aktivierungsorgans (13) eines in den Haltemitteln (6, 7) zu haltenden Insertionskopfes (12) mit mindestens einer ausklappbaren Infusionskanüle (11) und/- oder mindestens einer ausklappbaren Einstechspitze während dem Bewegen der Haltemittel (6, 7) mit dem darin bestimmungsgemäss gehaltenen Insertionskopf (12) von der Einsetzposition in die Bereitschaftsposition aufweist, zur Ermöglichung eines automatischen Ausklappens sämtlicher ausklappbarer Infusionskanülen (11) und Einstechspitzen des Insertionskopfes (12) beim Bewegen der Haltemittel (6, 7) mit dem bestimmungsgemäss daran gehaltenen Insertionskopf (12) von der Einsetzpositiön in die Bereitschaftsposition.

20. Insertionsvorrichtung nach Anspruch 19, **dadurch gekennzeichnet, dass** die Mittel (23) zur Bewirkung einer Verschiebung eines verschieblichen Aktivierungsorgans (13) eine Rampenfläche umfassen, an welcher ein querverschiebliches Aktivierungsorgan (13) eines in den Haltemitteln (6, 7) zu haltenden Insertionskopfes (12) mit mindestens einer ausklappbaren Infusionskanüle (11) und/oder mindestens einer ausklappbaren Einstechspitze während dem Bewegen der Haltemittel (6, 7) mit dem darin bestimmungsgemäss gehaltenen Insertionskopf (12) von der Einsetzposition in die Bereitschaftsposition anlaufen und dabei quer zur Bewegungsrichtung der Haltemittel (6, 7) und des Insertionskopfes (12) verschoben werden kann.

21. Insertionsvorrichtung nach einem der Ansprüche 19 bis 20, **dadurch gekennzeichnet, dass** die Mittel (23) zur Bewirkung einer Verschiebung eines verschieblichen Aktivierungsorgans (13) einen Hebelmechanismus (23) umfassen, mit welchem ein verschiebliches, insbesondere querverschiebliches Aktivierungsorgan (13) eines in den Haltemitteln (6, 7) zu haltenden Insertionskopfes (12) mit mindestens einer ausklappbaren Infusionskanüle (11) und/oder mindestens einer ausklappbaren Einstechspitze während dem Bewegen der Haltemittel (6, 7) mit dem darin bestimmungsgemäss gehaltenen Insertionskopf (12) von der Einsetzposition in die Bereitschaftsposition insbesondere quer zur Bewegungsrichtung der Haltemittel (6, 7) und des Insertionskopfes (12) verschoben werden kann.

22. Anordnung umfassend eine Insertionsvorrichtung nach einem der vorangehenden Ansprüche und einen in dieser aufgenommenen oder aufnehmbaren Insertionskopf (12) mit mindestens einer Infusionskanüle (11) und/oder mindestens einer Einstechspitze.

23. Anordnung nach Anspruch 22, **dadurch gekennzeichnet, dass** der Insertionskopf (12) ein Insertionskopf (12) mit mindestens einer ausklappbaren Infusionskanüle (11) und/oder mindestens einer ausklappbaren Einstechspitze ist, wobei sämtliche ausklappbaren Infusionskanülen (11) und Einstechspitzen des Insertionskopfes (12) im nicht-aktivierten Zustand, in welchem der Insertionskopf (12) zur Aufnahme in den in der Einsetzposition angeordneten Haltemitteln (6, 7) vorgesehen ist oder bereits in den in der Einsetzposition angeordneten Haltemitteln (6, 7) aufgenommen ist, eingeklappt sind und die Insertionsvorrichtung und der Insertionskopf (12) derartig ausgebildet sind, dass sämtliche ausklappbaren Infusionskanülen (11) und Einstechspitzen beim Bewegen der Haltemittel (6, 7) mit dem darin bestimmungsgemäss gehaltenen Insertionskopf (12) von der Einsetzposition in die Bereitschaftsposition ausgeklappt werden.

## Claims

1. Insertion device for an insertion head (12) with at least one infusion cannula (11) and/or with at least one puncturing tip for introduction into the body of a patient, comprising
a) at least one contact face (2) for placing the insertion device onto an application location on the body of the patient for application of the insertion head (12),
b) retention means (6, 7) by means of which the insertion head (12) that is to be applied is temporarily held on the insertion device,
c) drive means (8, 9) for effecting an insertion movement of the insertion head (12) relative to the contact face (2), in the longitudinal direction of an infusion cannula (11) or puncturing tip of the insertion head (12) ready for application, from a first position, in which the insertion head (12) ready for application is held by the retention means (6, 7) in such a way that all the infusion cannulas (11) and puncturing tips of the insertion head (12) are set back relative to the contact face (2), to a second position, in which all of the infusion cannulas (11) and puncturing tips of the insertion head (12) protrude substantially completely beyond the contact face (2), in order to permit introduction of all the infusion cannulas (11) and puncturing tips of the insertion head (12) into the body of the patient when the insertion movement is executed with the contact face (2) of the insertion device placed on the body,
wherein the drive means (8, 9) have at least one pretensionable energy-storing element (8) for providing the drive energy, and the retention means (6, 7) are positionable relative to the contact face (2) in an engage position and in a standby position and are formed in such a way that the insertion head (12) to be applied may be arranged on the retention means (6, 7) in the engage position in such a way that it is held by these, and the retention means (6, 7) may then be brought to the standby position starting from the engage position, with the insertion head (12) held on them, wherein on reaching the standby position, the retention means (6, 7) hold the insertion head (12) in the first position ready for application, wherein the insertion device is formed in such a way that, at the start of the insertion movement, the insertion head (12) is separated from the retention means (6, 7), such that it may execute at least the greatest part of the insertion movement free of the retention means (6, 7), and wherein the insertion device is moreover formed in such a way that the pretensionable energy-storing element (8) is pretensioned during the movement of the retention means (6, 7) from the engage position to the standby position.

2. Insertion device according to claim 1, **characterized in that** the insertion device is formed in such a way that, with the retention means (6, 7) arranged in the standby position, the insertion head (12) may be held by the retention means (6, 7) in the first position purely with a force fit, purely with a form fit, or with a combination of a force fit and form fit.

3. Insertion device according to claim 2, **characterized in that** the insertion device is formed in such a way that, with the retention means (6, 7) arranged in the engage position, the insertion head (12) may be held by the retention means (6, 7) purely with a form fit or with a combination of a force fit and form fit, and particularly wherein the form fit is cancelled during the movement of the retention means (6, 7), with the insertion head (12) held therein, from the engage position to the standby position.

4. Insertion device according to claim 2, **characterized in that** the insertion device is formed in such a way that, with the retention means arranged in the engage position, the insertion head (12) is held by the retention means (6, 7) purely with a force fit, and particularly wherein the force fit is reduced during the movement of the retention means (6, 7), with the insertion head (12) held therein, from the engage position to the standby position.

5. Insertion device according to one of the preceding claims, **characterized in that** the insertion device is formed in such a way that, at least during most of the insertion movement or during the entire insertion movement of the insertion head (12), the retention means (6, 7) remain unmoved relative to the contact face (2).

6. Insertion device according to one of the preceding claims, **characterized in that** the insertion device has manually activatable actuation means (6), particularly a slider element (6), a rotary knob (25) or a push button, by means of which the retention means (6, 7), with the insertion head (12) held thereon, may be brought manually by muscle force from the engage position to the standby position, with pretensioning of the energy-storing element (8).

7. Insertion device according to claim 6, **characterized in that** the insertion device comprises a housing (1), and the retention means (6, 7) are connected particularly rigidly to a slider element (6) which may be gripped by hand and may be moved, particularly displaced, relative to the housing (1), in order to move the retention means (6, 7) from the engage position to the standby position, with pretensioning of the energy-storing element (8).

8. Insertion device according to one of the preceding claims, **characterized in that** the insertion device is formed in such a way that its overall dimensions remain unchanged during the movement of the retention means (6, 7) from the engage position to the standby position.

9. Insertion device according to one of the preceding claims, **characterized in that** the insertion device is formed in such a way that the energy-storing element (8) may be repeatedly pretensioned, in order to permit repeated use of the insertion device for application of an insertion head (12).

10. Insertion device according to one of the preceding claims, **characterized in that** the insertion device is formed in such a way that, with the insertion head (12) located in the first position, the energy-storing element (8) may be made ready in the pretensioned state, and the insertion movement may then be triggered by actuation of at least one actuation member (3, 4), with increasing relaxation of the energy-storing element (8).

11. Insertion device according to claim 10, **characterized in that** the drive means (8, 9) comprise a thrust element (9) for transmitting the drive energy to the insertion head (12) to be applied and are formed in such a way that, by displacing the thrust element (9) counter to the direction of the insertion movement and subsequently locking it with locking means (15, 18) that may be released by the actuation members (3, 4), the energy-storing element (8) may be pretensioned and made ready in the pretensioned state.

12. Insertion device according to one of claims 10 and 11, **characterized in that** the insertion device has at least two actuation members (3, 4), which have to be actuated simultaneously in order to trigger the insertion movement, wherein particularly a first of the actuation members (3) is formed in such a way that it may be actuated by the contact face (2) of the insertion device being pressed onto the body of the patient, particularly in the direction of the insertion movement of the insertion head (12).

13. Insertion device according to claim 12, **characterized in that** the first actuation member (3) is formed as a slider-shaped or button-shaped element (3), which particularly forms all the contact faces (2).

14. Insertion device according to one of claims 12 and 13, **characterized in that** a second actuation member (4) is formed as a key-shaped or button-shaped element (4) which, when a user presses it with a finger tip, may be actuated particularly in a direction transverse, particularly perpendicular, to the intended direction in which the insertion device is pressed onto the body of the patient.

15. Insertion device according to one of claims 12 to 14, **characterized in that** the at least two actuation members (3, 4) are operatively connected to one another in such a way that, by actuating one actuation member (3), a blocking of the other actuation member (4) may be cancelled.

16. Insertion device according to one of claims 10 to 15, **characterized in that** the actuation member or the actuation members (3, 4) are formed in such a way that they may be actuated with one hand, in order to permit one-handed triggering of the insertion movement.

17. Insertion device according to one of claims 10 to 16, **characterized in that** the actuation members (3, 4) are formed in such a way that, when an actuating force ceases, they automatically go back to an unactuated state.

18. Insertion device according to one of the preceding claims, **characterized in that** the energy-storing element (8) is a helical spring, leg spring or leaf spring made of metal or plastic, a rubber spring element, or a pneumatic compression spring.

19. Insertion device according to one of the preceding claims, **characterized in that** the insertion device has means (23) for effecting a displacement, particularly a transverse displacement, of a displaceable actuation member (13) of an insertion head (12) to be held in the retention means (6, 7), with at least one deployable infusion cannula (11) and/or at least one deployable puncturing tip, during the movement of the retention means (6, 7), with the insertion head (12) held correctly therein, from the engage position to the standby position, in order to permit an automatic deployment of all the deployable infusion cannulas (11) and puncturing tips of the insertion head (12) during the movement of the retention means (6, 7), with the insertion head (12) held correctly thereon, from the engage position to the standby position.

20. Insertion device according to claim 19, **characterized in that** the means (23) for effecting a displacement of a displaceable actuation member (13) comprise a ramp surface on which a transversely displaceable actuation member (13) of an insertion head (12) to be held in the retention means (6, 7), with at least one deployable infusion cannula (11) and/or at least one deployable puncturing tip, may run during the movement of the retention means (6, 7), with the insertion head (12) held therein, from the engage position to the standby position and in so doing may be displaced transverse to the direction of movement of the retention means (6, 7) and of the insertion head (12).

21. Insertion device according to one of claims 19 and 20, **characterized in that** the means (23) for effecting a displacement of a displaceable actuation member (13) comprise a lever mechanism (23) by means of which a displaceable, particularly a transversely displaceable actuation member (13) of an insertion head (12) to be held in the retention means (6, 7), with at least one deployable infusion cannula (11) and/or at least one deployable puncturing tip, may be displaced, during the movement of the retention means (6, 7) with the insertion head (12) held correctly therein, from the engage position to the standby position, particularly transverse to the direction of movement of the retention means (6, 7) and of the insertion head (12).

22. Arrangement comprising an insertion device according to one of the preceding claims, and an insertion head (12) which is or may be received in the latter and which has at least one infusion cannula (11) and/or at least one puncturing tip.

23. Arrangement according to claim 22, **characterized in that** the insertion head (12) is an insertion head (12) with at least one deployable infusion cannula (11) and/or at least one deployable puncturing tip, all the deployable infusion cannulas (11) and puncturing tips of the insertion head (12) being folded inwards in the unactuated state, in which the insertion head (12) is intended to be received in the retention means (6, 7) arranged in the engage position or is already received in the retention means (6, 7) arranged in the engage position, and the insertion device and the insertion head (12) are formed in such a way that all the deployable infusion cannulas (11) and puncturing tips are deployed during the movement of the retention means (6, 7), with the insertion head (12) held correctly therein, from the engage position to the standby position.

## Revendications

1. Dispositif d'insertion pour une tête d'insertion (12) avec au moins une canule d'infusion (11) et/ou avec au moins une pointe à piquer pour la piqûre dans le corps d'un patient, comportant
a) au moins une surface de contact (2) pour le placement du dispositif d'insertion à un endroit d'application sur le corps du patient lors de l'application de la tête d'insertion (12),
b) des moyens de retenue (6, 7) pour le maintien provisoire de la tête d'insertion (12) à appliquer sur le dispositif d'insertion,
c) des moyens d'entraînement (8, 9) pour provoquer un mouvement d'insertion de la tête d'insertion (12) par rapport à la surface de contact (2) dans le sens longitudinal d'une canule d'infusion (11) ou pointe à piquer de la tête d'insertion (12) prête à l'application d'une première position, dans laquelle la tête d'insertion (12) prête à l'application est maintenue avec les moyens de retenue (6, 7) de telle sorte que toutes les canules d'infusion (11) et les pointes à piquer de la tête d'insertion (12) soient en retrait par rapport à la surface de contact (2), à une seconde position, dans laquelle toutes les canules d'infusion (11) et pointes à piquer de la tête d'insertion (12) dépassent essentiellement complètement de la surface de contact (2), pour permettre une piqûre de toutes les canules d'infusion (11) et pointes à piquer de la tête d'insertion (12) dans le corps du patient lors de la réalisation du mouvement d'insertion lorsque le dispositif d'insertion est placé avec la surface de contact (2) sur le corps,
les moyens d'entraînement (8, 9) présentant au moins un élément d'accumulation d'énergie (8) pouvant être précontraint pour mettre à disposition l'énergie d'entraînement et les moyens de retenue (6, 7) pouvant être positionnés par rapport à la surface de contact (2) dans une position d'insertion et dans une position en attente et sont configurés de telle sorte que la tête d'insertion (12) à appliquer dans la position d'insertion peut être disposée sur les moyens de retenue (6, 7) de telle sorte qu'elle soit maintenue par ceux-ci et les moyens de retenue (6, 7) pouvant être amenés ensuite en partant de la position d'insertion avec la tête d'insertion (12) maintenue dessus dans la position en attente, la tête d'insertion (12) étant maintenue lors de l'atteinte de la position en attente par les moyens de retenue (6, 7) de manière à être prête à l'application dans la première position, le dispositif d'insertion étant réalisé de telle sorte que la tête d'insertion (12) soit séparée au début du mouvement d'insertion des moyens de retenue (6, 7) de telle sorte que celle-ci puisse exécuter au moins la plus grande partie du mouvement d'insertion détachée des moyens de retenue (6, 7), et le dispositif d'insertion étant réalisé en outre de telle sorte que l'élément d'accumulation d'énergie (8) pouvant être précontraint soit précontraint lors du déplacement des moyens de retenue (6, 7) de la position d'insertion à la position en attente.

2. Dispositif d'insertion selon la revendication 1, **caractérisé en ce que** le dispositif d'insertion est réalisé de telle sorte que la tête d'insertion (12) puisse être maintenue lorsque les moyens de retenue (6, 7) sont disposés dans la position en attente dans la première position purement par adhérence, purement par correspondance de formes ou par adhérence et par correspondance de formes par les moyens de retenue (6, 7).

3. Dispositif d'insertion selon la revendication 2, **caractérisé en ce que** le dispositif d'insertion est réalisé de telle sorte que la tête d'insertion (12) puisse être maintenue lorsque les moyens de retenue (6, 7) sont disposés en position d'insertion purement par correspondance de formes ou par adhérence et par correspondance de formes par les moyens de retenue (6, 7) et en particulier la liaison par correspondance de formes étant supprimée lors du déplacement des moyens de retenue (6, 7) avec la tête d'insertion (12) maintenue dedans de la position d'insertion à la position en attente.

4. Dispositif d'insertion selon la revendication 2, **caractérisé en ce que** le dispositif d'insertion est réalisé de telle sorte que la tête d'insertion (12) puisse être maintenue lorsque les moyens de retenue sont disposés dans la position d'insertion purement par adhérence par les moyens de retenue (6, 7) et en particulier la liaison par adhérence étant réduite lors du déplacement des moyens de retenue (6, 7) avec la tête d'insertion (12) maintenue dedans de la position d'insertion à la position en attente.

5. Dispositif d'insertion selon l'une quelconque des revendications précédentes, **caractérisé en ce que** le dispositif d'insertion est réalisé de telle sorte que les moyens de retenue (6, 7) ne sont pas mobiles par rapport à la surface de contact (2) au moins pendant une grande partie du mouvement d'insertion ou pendant l'ensemble du mouvement d'insertion de la tête d'insertion (12).

6. Dispositif d'insertion selon l'une quelconque des revendications précédentes, **caractérisé en ce que** le dispositif d'insertion présente des moyens d'activation (6) actionnables manuellement, en particulier un élément de coulisseau (6), un bouton tournant (25) ou un bouton-poussoir, au moyen duquel les moyens de retenue (6, 7) avec la tête d'insertion (12) maintenue dessus peuvent être amenés manuellement par la force musculaire de la position d'insertion à la position en attente en précontraignant l'élément d'accumulation d'énergie (8).

7. Dispositif d'insertion selon la revendication 6, **caractérisé en ce que** le dispositif d'insertion comporte un boîtier (1) et les moyens de retenue (6, 7) sont reliés en particulier fixement à un élément de coulisseau (6) qui peut être saisi à la main et déplacé par rapport au boîtier (1), en particulier coulissé pour le déplacement des moyens de retenue (6, 7) de la position d'insertion à la position en attente en précontraignant l'élément d'accumulation d'énergie (8).

8. Dispositif d'insertion selon l'une quelconque des revendications précédentes, **caractérisé en ce que** le dispositif d'insertion est réalisé de telle sorte que ses dimensions globales restent inchangées lors du déplacement des moyens de retenue (6, 7) de la position d'insertion à la position en attente.

9. Dispositif d'insertion selon l'une quelconque des revendications précédentes, **caractérisé en ce que** le dispositif d'insertion est configuré de telle sorte que l'élément d'accumulation d'énergie (8) puisse être précontraint de manière répétée pour permettre une utilisation répétée du dispositif d'insertion pour l'application d'une tête d'insertion (12).

10. Dispositif d'insertion selon l'une quelconque des revendications précédentes, **caractérisé en ce que** le dispositif d'insertion est configuré de telle sorte que l'élément d'accumulation d'énergie (8) puisse être mis à disposition lorsque la tête d'insertion (12) se trouve dans la première position à l'état précontraint et le mouvement d'insertion peut être déclenché ensuite par l'actionnement d'au moins un organe d'actionnement (3, 4) sous détente croissante de l'élément d'accumulation d'énergie (8).

11. Dispositif d'insertion selon la revendication 10, **caractérisé en ce que** les moyens d'entraînement (8, 9) comportent un élément de propulsion (9) pour la transmission de la force d'entraînement à la tête d'insertion (12) à appliquer et sont configurés de telle sorte que l'élément d'accumulation d'énergie (8) puisse être précontraint en coulissant l'élément de propulsion (9) dans le sens opposé du mouvement d'insertion et en encliquetant celui-ci de manière contiguë avec des moyens d'encliquetage (15, 18) détachables par les organes d'actionnement (3, 4) et puisse être mis à disposition à l'état précontraint.

12. Dispositif d'insertion selon l'une quelconque des revendications 10 à 11, **caractérisé en ce que** le dispositif d'insertion présente au moins deux organes d'actionnement (3, 4) qui doivent être actionnés en même temps pour le déclenchement du mouvement d'insertion, en particulier un premier des organes d'actionnement (3) étant réalisé de telle sorte qu'il puisse être actionné par une pression du dispositif d'insertion avec la surface de contact (2) sur le corps du patient, en particulier en direction du mouvement d'insertion de la tête d'insertion (12).

13. Dispositif d'insertion selon la revendication 12, **caractérisé en ce que** le premier organe d'actionnement (3) est réalisé comme un élément (3) en forme de coulisseau ou de bouton-poussoir qui forme en particulier toutes les surfaces de contact (2).

14. Dispositif d'insertion selon l'une quelconque des revendications 12 à 13, **caractérisé en ce qu'**un second organe d'actionnement (4) est réalisé comme un élément (4) en forme de touche ou de bouton qui peut être actionné par sollicitation de pression par un bout de doigt de l'utilisateur, en particulier dans une direction transversalement en particulier perpendiculairement au sens de pression selon les dispositions du dispositif d'insertion sur le corps du patient.

15. Dispositif d'insertion selon l'une quelconque des revendications 12 à 14, **caractérisé en ce que** les au moins deux organes d'actionnement (3, 4) sont reliés opérationnellement l'un à l'autre de telle sorte que par l'actionnement d'un organe d'actionnement (3), un blocage de l'autre organe d'actionnement (4) puisse être supprimé.

16. Dispositif d'insertion selon l'une quelconque des revendications 10 à 15, **caractérisé en ce que** l'organe d'actionnement ou les organes d'actionnement (3, 4) sont réalisés de telle sorte que ceux-ci puissent être actionnés à une main pour permettre un déclenchement à une seule main du mouvement d'insertion.

17. Dispositif d'insertion selon l'une quelconque des revendications 10 à 16, **caractérisé en ce que** les organes d'actionnement (3, 4) sont réalisés de telle sorte qu'ils passent automatiquement de nouveau dans un état non actionné lors de la suppression d'une force d'actionnement.

18. Dispositif d'insertion selon l'une quelconque des revendications précédentes, **caractérisé en ce que** l'élément d'accumulation d'énergie (8) est un ressort à boudin, à branches, ou à lames en métal ou en matière plastique, un élément de ressort en caoutchouc ou un ressort de pression de gaz.

19. Dispositif d'insertion selon l'une quelconque des revendications précédentes, **caractérisé en ce que** le dispositif d'insertion présente des moyens (23) pour provoquer un déplacement, en particulier un déplacement transversal, d'un organe d'activation mobile (13) d'une tête d'insertion (12) à maintenir dans les moyens de retenue (6, 7) avec au moins une canule d'infusion (11) escamotable et/ou au moins une pointe à piquer escamotable pendant le déplacement des moyens de retenue (6, 7) avec la tête d'insertion (12) maintenue dedans selon les dispositions de la position d'insertion à la position en attente, pour permettre un escamotage automatique de toutes les canules d'infusion (11) et pointes à piquer escamotables de la tête d'insertion (12) lors du déplacement des moyens de retenue (6, 7) avec la tête d'insertion (12) maintenue dessus selon les dispositions de la position d'insertion à la position en attente.

20. Dispositif d'insertion selon la revendication 19, **caractérisé en ce que** les moyens (23) pour provoquer un déplacement d'un organe d'activation (13) mobile comportent une surface de rampe, sur laquelle un organe d'activation (13) mobile transversalement d'une tête d'insertion (12) à maintenir dans les moyens de retenue (6, 7) avec au moins une canule d'infusion (11) escamotable et/ou au moins une pointe à piquer escamotable peut démarrer pendant le déplacement des moyens de retenue (6, 7) avec la tête d'insertion (12) maintenue dedans selon les dispositions de la position d'insertion à la position en attente et être déplacé transversalement au sens de déplacement des moyens de retenue (6, 7) et de la tête d'insertion (12).

21. Dispositif d'insertion selon l'une quelconque des revendications 19 à 20, **caractérisé en ce que** les moyens (23) pour provoquer un déplacement d'un organe d'activation (13) mobile comportent un mécanisme à levier (23), avec lequel un organe d'activation (13) mobile, en particulier mobile transversalement d'une tête d'insertion (12) à maintenir dans les moyens de retenue (6, 7) avec au moins une canule d'infusion (11) escamotable et/ou au moins une pointe à piquer escamotable peut être déplacé pendant le déplacement des moyens de retenue (6, 7) avec la tête d'insertion (12) maintenue dedans selon les dispositions de la position d'insertion à la position en attente en particulier transversalement au sens de déplacement des moyens de retenue (6, 7) et de la tête d'insertion (12).

22. Ensemble comportant un dispositif d'insertion selon l'une quelconque des revendications précédentes et une tête d'insertion (12) logée ou pouvant être logée dans celui-ci avec au moins une canule d'infusion (11) et/ou au moins une pointe à piquer.

23. Ensemble selon la revendication 22, **caractérisé en ce que** la tête d'insertion (12) est une tête d'insertion (12) avec au moins une canule d'infusion (11) escamotable et/ou au moins une pointe à piquer (11) escamotable, toutes les canules d'infusion (11) et pointes à piquer escamotables de la tête d'insertion (12) étant escamotées à l'état non activé, dans lequel la tête d'insertion (12) est prévue pour le logement dans les moyens de retenue (6, 7) disposés dans la position d'insertion ou est déjà logée dans les moyens de retenue (6, 7) disposés dans la position d'insertion et le dispositif d'insertion et la tête d'insertion (12) étant réalisés de telle sorte que toutes les canules d'infusion (11) escamotables et pointes à piquer soient escamotées lors du déplacement des moyens de retenue (6, 7) avec la tête d'insertion (12) maintenue dedans selon les dispositions de la position d'insertion à la position en attente.
